# EUROPEAN PATENT APPLICATION

(11) **EP 4 458 985 A1**
(43) Date of publication of application: **06.11.2024**
(21) Application number: 22914814.3
(22) Date of filing: 27.12.2022
(51) Int. Cl.: C12Q 1/686, C12N 15/11, C12M 1/34, C12M 1/38

(54) **METHOD FOR DETECTING MULTIPLE TARGET NUCLEIC ACIDS**

(30) Priority: 27.12.2021 CN 202111617233
(71) Applicant: Maccura Biotechnology Co., Ltd., Chengdu Sichuan 611731 (CN)
(72) Inventor: ZHAO, Yuhang, Chengdu, Sichuan 611731 (CN); HU, Waner, Chengdu, Sichuan 611731 (CN); WANG, Haixin, Chengdu, Sichuan 611731 (CN); HUANG, Qiuping, Chengdu, Sichuan 611731 (CN)
(74) Representative: Laine IP Oy
(86) International application number: PCT/CN2022/142473
(87) International publication number: WO 2023/125562

(57) **Abstract**

The present invention relates to the field of molecular biology, in particular to a method for detecting multiple target nucleic acids in a single sample container. The present invention particularly relates to a method for multiplex detection based on digital PCR. The method comprises: mixing a primer-probe composition, a sample to be detected and an amplification reagent to obtain a reaction system; placing the reaction system in a condition that allows a nucleic acid polymerase to perform hybridization and extension reaction, so as to obtain a reaction product; and performing signal acquisition on the reaction product. The detection method of the present invention can realize detection of multiple target nucleic acids, is easy to operate, and can save time.

## Description

### Cross-reference of related applications

The present application claims the priority of the Chinese patent application No. CN 202111617233.2 filed on December 27, 2021 and entitled "primer probe for detection, primer probe set, and application thereof", the entirety of which is incorporated herein by reference.

### Field of Technology

The present invention relates to the field of molecular biology, in particular to a method for detecting multiple target nucleic acids in a single sample container.

### Background

Polymerase chain reaction (PCR) is a molecular biological technique for enzymatic replication of DNA without using a living organism. PCR is commonly used in medical and biological research laboratories to undertake a variety of tasks, such as diagnosis of infectious diseases, gene cloning, phenotype identification of experimental animals, transcriptome research, detection of genetic diseases, identification of genetic fingerprints, and paternity testing. Because of unrivaled abilities of PCR to replicate and be precise, it is considered by molecular biologists to be a preferred method for nucleic acid detection. In the late 1990s, American ABI company launched Real Time Quantitative PCR (qPCR) technique and related products, which further developed PCR into a highly-sensitive, highly-specific and accurate quantitative nucleic acid sequence analysis technique.

Digital PCR (dPCR) is a technique for absolute quantification of nucleic acid molecules that utilizes the principle of limiting dilution to distribute one fluorescence quantitative PCR reaction system into thousands of individual nanoliter microreactors so that each microreactor contains no, or one or more copies of a target nucleic acid molecule (DNA target) to be simultaneously subj ected to single-molecule template PCR amplification. Different from fluorescence quantitative PCR, which collects fluorescence at each amplification cycle, digital PCR collects a fluorescence signal of each reaction unit independently at the end of amplification, and finally works out an original number of copies or concentration of a target molecule through the principle of Poisson distribution and the ratio of positive/negative reaction units.

Compared to fluorescence quantitative PCR, digital PCR may perform accurate absolute quantitative detection without relying on a Ct value and a standard curve, and has the advantages of high sensitivity as well as high accuracy. Since digital PCR only determines two amplification states of "yes/no" when performing result interpretation, there is also no need to detect an intersection of a fluorescence signal and a set threshold line, and it is completely independent of identification of the Ct value, so that the impact of amplification efficiency on digital PCR reaction and result interpretation is greatly reduced, and the tolerance to a PCR reaction inhibitor is greatly improved. In addition, the process of distributing the reaction system in a digital PCR experiment may greatly reduce the concentration of a background sequence that competes with a target sequence locally, and thus digital PCR is particularly suitable for detection of a rare mutation in a complex background, and is currently mostly applied in liquid biopsies to realize detection of rare mutation markers in peripheral blood of tumor patients.

In order to realize multiplex detection of digital PCR, a common method is to design one kind of probe for each target, and realize detection of different targets through different fluorescence of different probes. However, too many classes of probes are not only costly, but also have a high fluorescence background, which reduces the sensitivity of detection.

### Summary

In order to solve the above problems, the present invention provides a method for detecting multiple target nucleic acids, including the following contents:
mixing a primer-probe composition, a sample to be detected and an amplification reagent to obtain a reaction system; the amplification reagent comprising a nucleic acid polymerase and dNTPs;
placing the reaction system in a condition that allows the nucleic acid polymerase to perform hybridization and extension reaction, so as to obtain a reaction product;
placing the reaction product at n different signal acquisition temperatures for a total of n signal acquisitions with each signal acquisition temperature for one signal acquisition; each signal acquisition comprising at least one signal channel acquisition;
analyzing whether there is a difference between signals obtained from two adjacent signal channel acquisitions under a same signal channel to determine presence or absence of the target nucleic acids in the sample to be detected; and/or
analyzing presence or absence of a signal obtained from a signal channel acquisition with a maximum signal acquisition temperature under the same signal channel to determine the presence or absence of the target nucleic acids in the sample to be detected;
wherein n is an integer ≥ 2, and n ≤ a number of classes of the target nucleic acids.

By adopting above detection method, the multiple target nucleic acids can be detected (at least octuple reaction may be realized on digital PCR). The realization of multiplex detection not only relies on fluorescence channels, but also uses different melting temperatures in the same fluorescence channel (i.e., the same signal channel), and different targets may be distinguished by the presence or absence of fluorescence through a finite number of photographs, which has low fluorescence recognition requirements for digital PCR, is easy to operate and saves time.

In certain embodiments, one signal acquisition is performed at each signal acquisition temperature; and n different signal acquisition temperatures are used, and thus n signal acquisitions are performed. In certain embodiments, n refers to the number of signal acquisitions; and n ≤ the number of classes of the target nucleic acids, that is, the number of signal acquisitions ≤ the number of classes of the target nucleic acids.

In certain embodiments, numbers of classes of target nucleic acids contained in the signals obtained from the two adjacent signal channel acquisitions under the same signal channel differ by one at most; in certain embodiments, the numbers of classes of the target nucleic acids contained in the signals obtained from the two adjacent signal channel acquisitions under the same signal channel differ by one; and in certain embodiments, the signal obtained from the signal channel acquisition with the maximum signal acquisition temperature contains at most one kind of target nucleic acid. In certain embodiments, the "one kind of target nucleic acid" further includes one class of target nucleic acid that is classified but not typed.

In certain embodiments, "signal acquisition" includes the following operations: performing a first signal acquisition on the reaction product at a first temperature, heating up to a second temperature for a second signal acquisition, continuing heating up to a third temperature for a third signal acquisition, continuing heating up and signal acquisition, until all of the target nucleic acids in the sample to be detected are detected. It may also include operations for signal acquisitions in continuous cooling: for example, performing a first signal acquisition on the reaction product at a first temperature, cooling down to a second temperature for a second signal acquisition, continuing cooling down to a third temperature for a third signal acquisition, continuing cooling down and signal acquisition, until all of the target nucleic acids in the sample to be detected are detected.

In certain embodiments, there is only one kind of probe in the primer-probe composition (one kind of probe refers to one class of probe that is modified with the same detection labels or fluorophores and may have the same or different base sequences), and thus each signal acquisition only includes one signal channel acquisition, that is, each signal acquisition refers to one signal acquisition under a certain signal channel (fluorescence channel). At this point, two adjacent signal channel acquisitions refer to two adjacent signal acquisitions, the numbers of classes of target nucleic acids contained in signals obtained from two adjacent signal channel acquisitions differ by one, and thus the number of signal acquisitions is equal to the number of classes of the target nucleic acids. For example, the sample to be detected contains three kinds of target nucleic acids, under a certain fluorescence channel, a first signal acquisition is performed on the reaction product at a first temperature (equivalent to a signal channel acquisition) to obtain a signal, which contains three kinds of targets, the reaction product heats up to a second temperature for a second signal acquisition to obtain a signal, which contains two kinds of targets, and the reaction product continues heating up to a third temperature for a third signal acquisition to obtain a signal, which contains one kind of target. The presence of the target nucleic acids may be determined by comparing the signals of the two adjacent signal acquisitions. For example, if part of the signals disappears during the second signal acquisition compared to the first signal acquisition, the disappeared signal is a first target; if the other part of the signals disappears during the third signal acquisition compared to the second signal acquisition, the other part of the signals that has disappeared is a second target, the signal that is still presented in the third signal acquisition is a third target. As such, it is possible to determine whether or not all of the three targets are presented. In addition, it is also possible to determine the content of each kind of target according to the number of the disappeared/presented signals.

In certain embodiments, there are two kinds of probes in the primer-probe composition (two kinds of probes refer to two classes of probes that are modified with different detection labels or fluorophores and have different base sequences), and thus at least one signal acquisition includes two signal channel acquisitions. Two signal channel acquisitions refer to one signal acquisition under each of two signal channels (fluorescence channels). According to the design of the primer-probe composition, a person skilled in the art may predict the melting temperature of the reaction product and further predict the temperature required for the reaction product representing the target nucleic acid to generate a detectable signal, and a fluorescence channel in which it is located. Therefore, according to the design of the primer-probe composition, a person skilled in the art may select a suitable signal acquisition temperature and a suitable signal channel (fluorescence channel) for a signal acquisition. As such, in the embodiment, analysis may be simultaneously performed in single-tube reaction in two dimensions of a fluorescence channel and a melting temperature. That is, by using the same fluorescence channel, different targets may be detected through different signal acquisition temperatures; or by using different fluorescence channels, the detection of the classes of the targets which are a product of the number of the fluorescence channels and signal acquisition temperature characteristics can be achieved.

In certain embodiments, the amplification reagent may further include some reagents that promote PCR reaction, such as KCl, MgCl₂, Tris-HCl, dithiothreitol (DTT) and (NH₄)₂SO₄. In certain embodiments, in addition to a polymerase with polymerization activity, the amplification reagent further includes other enzymes that act on nucleic acids, such as exonuclease and endonuclease.

In certain embodiments, two adjacent signal channel acquisitions under the same signal channel means that signal acquisition temperatures used for the two signal channel acquisitions are close. For example, in certain embodiments, under the same signal channel, the reaction product is placed at a first temperature for a first signal channel acquisition, heats up to a second temperature for a second signal channel acquisition, and continues heating up to a third temperature for a third signal channel acquisition. Thus, the first signal channel acquisition and the second signal channel acquisition are two adjacent signal channel acquisitions, the second signal channel acquisition and the third signal channel acquisition are two adjacent signal channel acquisitions, but the first signal channel acquisition and the third signal channel acquisition are not two adjacent signal channel acquisitions.

In certain embodiments, signal acquisition temperatures used for two adjacent signal channel acquisitions differ by 4°C or above to realize a difference of one between the numbers of classes of target nucleic acids contained in signals obtained from the two adjacent signal channel acquisitions under the same signal channel, avoiding the situation in which there is no difference in the target nucleic acid signals obtained from the two adjacent signal channel acquisitions, thereby reducing the number of signal channel acquisitions and simplifying operation steps.

In certain embodiments, the signal acquisition temperature is 0-95°C. The selection of the signal acquisition temperature may be determined by a person skilled in the art according to the actual situation of the designed primer-probe composition. For example, through the design of the primer-probe composition, a person skilled in the art may predict that the reaction product representing the target nucleic acid has a melting temperature of T. If it is desired to detect the signal of the target nucleic acid, the signal acquisition temperature ≤ T; and if it is not desired to detect the signal of the target nucleic acid, the signal acquisition temperature > T.

In certain embodiments, each signal acquisition includes m signal channel acquisitions; and m refers to the number of classes of detection labels of a probe in the primer-probe composition. In order to facilitate programming design of a detection instrument, in certain embodiments, if there are two kinds of probes in the primer-probe composition, each signal acquisition refers to two signal channel acquisitions under two signal channels. In certain embodiments, there are five kinds of targets in the sample to be detected. The primer-probe composition for the five kinds of targets contains two kinds of probes, with the first probe detecting three kinds of targets and the second probe detecting two kinds of targets. Thus, only three signal acquisitions are required for the reaction product, and each signal acquisition refers to two signal channel acquisitions under two signal channels. As such, there is a situation in which three signal acquisitions are performed under a certain signal channel, but there are only two kinds of corresponding targets, and thus there is a situation in which the numbers of classes of target nucleic acids contained in signals obtained from two signal acquisitions with adjacent signal acquisition temperatures differ by zero. In these embodiments, the number of signal acquisitions is set in terms of the detection channel that detects the maximum number of targets, which realizes that the number of signal acquisitions < the number of classes of target nucleic acids, and reduces the number of signal acquisitions. Although it does not fully meet the situation that other signal channels have the minimum number of signal acquisitions, it is easier to set up an instrument program, and a person skilled in the art may choose according to actual needs. In certain embodiments, a person skilled in the art, according to the design of the primer-probe composition, chooses not to perform a signal acquisition in channels that do not have an expected target, thereby reducing the number of signal acquisitions and simplifying operation steps.

In certain embodiments, the above method for detecting multiple target nucleic acids refers to a digital PCR detection method for multiple target nucleic acid. Before the placing the reaction system in a condition that allows the nucleic acid polymerase to perform hybridization and extension reaction, the method further includes: distributing the reaction system into 500 or more reaction units, with each reaction unit containing one target nucleic acid of the sample to be detected or containing no target nucleic acids of the sample to be detected. In certain embodiments, the signal acquisitions refer to acquisitions of fluorescence signals by means of a camera. In certain embodiments, the signal channel acquisitions refer to acquisitions of the fluorescence signals by means of the camera under a fluorescence signal channel.

In certain embodiments, the primer-probe composition, the sample to be detected (containing two kinds of targets) and the amplification reagent are mixed to obtain a reaction system, and the reaction system is distributed into 500 or more reaction units to form countless small droplets, each of which contains at most one target nucleic acid of the sample to be detected but contains necessary components suitable for amplification of nucleic acids, e.g., the primer-probe composition and amplification reagents such as the nucleic acid polymerase. All the small droplets are placed into the condition that allows the nucleic acid polymerase to perform hybridization and extension reaction (in certain embodiments, i.e., a PCR amplification condition) to obtain a reaction product (amplification product), which undergoes a first photograph at a first temperature, part of the small droplets shines, and a positive signal with two kinds of targets is obtained. The reaction product heats up to a second temperature for a second photograph, and it is found that certain small droplets undergoing the first photograph do not shine, and certain small droplets still shine. Thus, the small droplets that still shine in the second photograph serve as first targets, and the small droplets that do not shine in the second photograph but shine in the first photograph serve as second targets. Specific classes of the two kinds of targets may be analyzed according to temperatures and fluorescence channels used in the two photographs. The number of the first targets is obtained by analyzing the number of the shining small droplets in the second photograph. The number of the second targets may be obtained by analyzing a difference value between the numbers of the shining small droplets in the first photograph and the second photograph.

In certain embodiments, the condition that allows the nucleic acid polymerase to perform hybridization and extension reaction includes: initial denaturation at about 85°C to about 105°C for 0 to about 15 min; denaturation at about 85°C to about 105°C for about 1 to about 60 s, and annealing and extending at about 40°C to about 75°C for about 3 to about 90 s, for 20 to 60 cycles; preferably, when a target in the sample to be detected is RNA, the amplification reagent further includes a reverse transcriptase, and a reaction condition for a first PCR amplification of the reaction system includes: reverse transcription at about 30 to about 65°C for about 2 to about 30 min; initial denaturation at about 85°C to about 105°C for 0 to about 15 min; denaturation at about 85°C to about 105°C for about 1 to about 60 s, and annealing and extending at about 40°C to about 75°C for about 3 to about 90 s, for 20 to 60 cycles.

In certain embodiments, the above condition that allows the nucleic acid polymerase to perform hybridization and extension reaction includes: initial denaturation at about 85°C to about 105°C for 0 to about 15 min; denaturation at about 85°C to about 105°C for about 2 to about 60 s, and annealing and extending at about 40°C to about 75°C for about 10 to about 90 s, for 20 to 60 cycles. In certain embodiments, when a target nucleic acid in the sample to be detected contains RNA, the amplification reagent further includes a reverse transcriptase, and the condition that allows the nucleic acid polymerase to perform hybridization and extension reaction includes: reverse transcription at about 30 to about 65°C for about 2 to about 30 min; initial denaturation at about 85°C to about 105°C for 0 to 15 min; denaturation at 85°C to105°C for 2 to 60 s, and annealing and extending at 40°C to 75°C for 10 to 90 s, for 20 to 60 cycles.

In certain embodiments, in the method for detecting multiple target nucleic acids, the above primer-probe composition includes a first probe and a first primer mixture; the first primer mixture includes at least two kinds of primer sets, and the different kinds of primer sets specifically bind to different kinds of target nucleic acids respectively. The primer sets in the first primer mixture specifically bind to the corresponding target nucleic acids to generate a pre-product, the pre-product contains a single-stranded pre-product specifically binding to the first probe, and the single-stranded pre-product specifically binds to the first probe and extends by >_ 0 base to form a double-stranded product, and formation of the double-stranded product causes a detectable signal change.

In certain embodiments, the different kinds of primer sets in the first primer mixture and the corresponding target nucleic acids generate different single-stranded pre-products, the different single-stranded pre-products and the first probe form different double-stranded products, and the different double-stranded products have different melting temperatures. In certain embodiments, the melting temperatures of the different double-stranded products differ by 4°C or above.

The first probe refers to one class of probe that is modified with the same detection labels and may have the same or different base sequences. That is, the number of classes of probes is determined according to the number of classes of detection labels thereof. In certain embodiments, a signal generated from one kind of probe may undergo a signal acquisition in one detection channel (or a fluorescence channel); and signals generated from two kinds of probes may undergo signal acquisitions in two different detection channels. In certain embodiments, the same detection labels do not mean that detection groups for modification are exactly the same. For example, the detection groups include fluorophores and quenchers. Signals generated from probes may also undergo signal acquisitions in one detection channel (or a fluorescence channel) as long as the fluorophores are the same.
"at least two kinds of primer sets" refers to at least two classes of primer sets. Each primer set is specific to a different kind of target nucleic acid, that is, each kind of primer set can specifically bind to a corresponding target nucleic acid. In certain embodiments, one kind of primer set can specifically bind to one class of non-typed target nucleic acids all, and thus this class of non-typed target nucleic acids may also be considered as one kind of target nucleic acid.

In certain embodiments, a primer set belongs to a double-primer case, i.e., including a pair of upstream and downstream primers specific to the same target nucleic acid. In certain embodiments, a primer set belongs to a single-primer case, i.e., only containing one kind of primer specific to a certain target nucleic acid. The primer can specifically bind to the corresponding target nucleic acid but cannot specifically bind to other kinds of target nucleic acids.

In certain embodiments, a common primer may be presented in different kinds of primer sets. For example, the same primer is included in two kinds of primer sets. The same kind of primer refers to sequences of primers being exactly the same.

In this embodiment, a first single-stranded pre-product generated from a primer set specific to a certain target nucleic acid does not extend after specifically binding to a probe (i.e., extending by 0 base). A person skilled in the art may, according to actual demands, achieve the above effect of "specifically binding but not extending" by designing a primer sequence in the primer set and/or a sequence of the probe. For example, it is designed in such a way that the first single-stranded pre-product generated from the primer set bound to a 5' end of the probe, or it is designed in such a way that a 3' end of the first single-stranded pre-product generated from the primer set contains a region that is not complementarily paired with the probe.

A melting temperature of an oligonucleotide is related to factors such as the length and composition of the oligonucleotide. A person skilled in the art may adjust the melting temperature of the oligonucleotide according to actual needs. For example, a higher melting temperature may be obtained by increasing the GC content of the oligonucleotide, making the length of the oligonucleotide longer. Therefore, a person skilled in the art may, according to actual situations, adjust sequence compositions of primers and/or probes to make the different primers to form different single-stranded pre-products. The different single-stranded pre-products specifically bind to the first probe at different locations, thereby forming different double-stranded products with different melting temperatures. In other words, a person skilled in the art may, according to actual situations, make the double-stranded products representing different targets different, and the different double-stranded products have the different melting temperatures, so that signals of different target nucleic acids are obtained under different signal acquisition temperatures. In certain embodiments, the melting temperatures of the different double-stranded products representing the different targets may differ by 4°C or above through the design of primer probes.

In certain embodiments, the primer-probe composition further includes a second probe and a second primer mixture; the second probe and the first probe have different base sequences and are modified with different detection labels; and the second primer mixture includes at least one kind of primer set, and different kinds of primer sets specifically bind to different kinds of target nucleic acids respectively.

The number of classes of probes is determined according to the number of classes of detection labels thereof. Different kinds of probes need to perform signal acquisitions under different detection channels due to different detection labels. In certain embodiments, different detection labels do not mean that detection groups for modification are completely different. For example, the detection groups include fluorophores and quenchers. Signals generated from two kinds of probes may also undergo signal acquisitions in two detection channels (or fluorescence channels) as long as the fluorophores are different (the quenchers may be the same or different). In certain embodiments, the increase in the number of classes of probes may realize signal detection of different target nucleic acids under different channels, and thus may realize more multiplex detection of the target nucleic acids.

In the embodiment, the presence of the "first probe" and the "second probe" is not intended to define the role, nor is it intended to define that there are only two kinds of probes in the embodiment. Here, it is only intended to distinguish that the "first probe" and the "second probe" belong to different classes of probes, and the two kinds of probes are modified with different detection labels and have different base sequences. In certain embodiments, in order to detect more classes of target nucleic acids, the primer-probe composition for target nucleic acid detection further includes a third probe and a third primer mixture, a fourth probe and a fourth primer mixture, a fifth probe and a fifth primer mixture, a sixth probe and a sixth primer mixture, or more probes and primer mixtures. Different probes are modified with different detection labels and have different base sequences; and primer sets in different primer mixtures are also different. In other words, in order to realize more multiplex detection of the target nucleic acids, a person skilled in the art may design more kinds of primer sets corresponding to the target nucleic acids and more kinds of probes corresponding to the primer sets as needed.

In certain embodiments, the primer sets in the second primer mixture specifically bind to the corresponding target nucleic acids to generate a pre-product, the pre-product contains a single-stranded pre-product specifically binding to the second probe, and the single-stranded pre-product specifically binds to the second probe and extends by >_ 0 base to form a double-stranded product, and formation of the double-stranded product causes a detectable signal change.

In certain embodiments, the different kinds of primer sets in the second primer mixture and the corresponding target nucleic acids generate different single-stranded pre-products, the different single-stranded pre-products and the second probe form different double-stranded products, and the different double-stranded products have different melting temperatures. In certain embodiments, the melting temperatures of the different double-stranded products differ by 4°C or above.

In certain embodiments, in the primer-probe composition, the first probe or the second probe is a sequence that does not specifically bind to any target nucleic acids, and includes a probe signal detection region (H), and sequences of the probe signal detection regions (H) of the different probes are different from each other; each primer set includes a first primer and a second primer, and the first primer contains target sequence binding regions 1; the second primer contains primer signal detection regions (h) and target sequence binding regions 2, and the primer signal detection regions (h) are located at 5' ends of the target sequence binding regions 2; each primer signal detection region (h) is a sequence that does not specifically bind to any target nucleic acids and partially or wholly identical to the probe signal detection region (H) of the corresponding probe; and sequences of the primer signal detection regions (h) of the second primers in the different primer sets are different from each other.

In certain embodiments, the target sequence binding regions 1 and the target sequence binding regions 2 specifically bind to different locations of the target nucleic acids respectively.

In certain embodiments, primer signal detection region (h) with a sequence that is partially or wholly identical to the probe signal detection region (H) of the corresponding probe means that reverse complementary sequences (h') of the primer signal detection regions (h) can specifically bind to part or all of the sequences of the probe signal detection regions (H) of the probes.

In certain embodiments, the first primer and the second primer specifically bind to the target nucleic acids to generate a pre-product, and the pre-product contains a single-stranded pre-product with the reverse complementary sequences (h') of the primer signal detection regions (h). The reverse complementary sequences (h') of the single-stranded pre-product specifically bind to the probe signal detection regions (H) of the probes and extend by >_ 0 base to form a double-stranded product, and formation of the double-stranded product causes a detectable signal change.

In certain embodiments, the sequences of the primer signal detection regions (h) of the second primers in the different primer sets are different from each other. That is, the sequences of the primer signal detection regions (h) of the second primers in the different primer sets have different bases and/or lengths, so that reverse complementary sequences (h') of different single-stranded pre-products are different. The different single-stranded pre-products specifically bind to the probes and extend by 0 or more bases to form different double-stranded products, and the melting temperatures of the different double-stranded products can be separated from each other; alternatively, the different single-stranded pre-products specifically bind to different kinds of probes respectively and extend by >_ 0 base to form different double-stranded products, the melting temperatures of the different double-stranded products cannot be separated from each other, however, since the different double-stranded products have different kinds of probes, these double-stranded products may be separated through different detection channels. That is, the different kinds of target nucleic acids may be distinguished through the melting temperatures and/or the detection channels to realize multiplex detection.

In certain embodiments, the primer signal detection regions (h) of the second primer and the target sequence binding regions 2 are spaced by 0-20 bases.

In some implementations of the present invention, as for the above primer-probe composition for target nucleic acid detection, the first probe or the second probe further includes a primer anchoring region (A'); the first primer of the at least one kind of primer set in the first primer mixture or the second primer mixture further includes a probe anchoring region (A), and the probe anchoring region (A) is located at a 5' end of the target sequence binding region 1; and the probe anchoring region (A) does not specifically bind to any target nucleic acids but specifically binds to the primer anchoring region (A').

The first primer and the second primer in the above primer sets specifically bind to the target nucleic acids to generate a pre-product, and the pre-product contains a single-stranded pre-product with the probe anchoring regions (A) and the reverse complementary sequences (h') of the primer signal detection regions (h). The probe anchoring regions (A) and the reverse complementary sequences (h') of the single-stranded pre-product specifically bind to the primer anchoring regions (A') of the probes and the probe signal detection regions (H) respectively and extend by >_ 0 base to form a double-stranded product, and formation of the double-stranded product causes a detectable signal change.

By adjusting the probe anchoring regions (A) of the first primers in the different primer sets and/or sequence compositions and/or lengths of the primer signal detection regions (h) of the second primers in the different primer sets, the different primer sets and the target nucleic acids thereof generate different single-stranded pre-products. The different single-stranded pre-products specifically bind to the probes to generate different annealing temperatures, and specifically bind to the probes and extend by >_ 0 base to form double-stranded products with different melting temperatures, so that the lower an annealing temperature required for a single-stranded pre-product generated from a certain kind of primer set and a target nucleic acid thereof specifically binding to a probe, the higher a melting temperature of a double-stranded product formed by the single-stranded pre-product specifically binding to the probe and extending by >_ 0 base.

In certain embodiments, the target sequence binding regions 1 of the first primers in the different primer sets have different sequences; the probe anchoring regions (A) of the first primers in the different primer sets may be the same or different; and preferably, the probe anchoring regions (A) of the first primer and the target sequence binding regions 1 are spaced by 0-20 bases.

In some implementations of the present invention, as for the above primer-probe composition for target nucleic acid detection, in the first primer mixture or the second primer mixture, the second primer of at most one kind of primer set further includes an extension blocking region (M), the extension blocking region (M) is located at a 5' end of the primer signal detection region (h), and neither the extension blocking region (M) nor a complementary sequence thereof specifically binds to any probes or any target nucleic acids.

The above first primers and the second primer containing the extension blocking region (M) specifically bind to target nucleic acids respectively to generate a pre-product, and the pre-product contains a single-stranded pre-product with the reverse complementary sequence (h') of the primer signal detection region (h). The reverse complementary sequence (h') of the single-stranded pre-product specifically binds to the probe and extends by 0 base to form a double-stranded product (due to the presence of the extension blocking region, the single-stranded pre-product specifically binds to the probe and does not extend), thereby causing the probe to generate a detectable signal change.

"First primer mixture", "second primer mixture", etc. are only used for the purpose of description to distinguish defined objects. Each of them represents one set of primer mixtures that specifically binds to probes. The primer mixtures include a plurality kinds of primer sets, and reverse complementary sequences (h') of primer signal detection regions (h) contained in second primers in the primer sets each may specifically bind to the same kind of probe. However, the primer sets in the "first primer mixture" and the "second primer mixture" specifically bind to different kinds of probes.

In certain embodiments, in one set of primer mixtures that specifically binds to the same kind of probe, the second primer of at most one kind of primer set contains an extension blocking region (M). The kind of primer set specifically binds to a target nucleic acid to generate a single-stranded pre-product, which requires a maximum annealing temperature for specifically binding to a probe, and specifically binds to the probe without extending to generate a double-stranded product with a minimum melting temperature.

In the present invention, "first primer" and "second primer" are only used for the purpose of description to distinguish the defined objects, instead of defining the order or primary and secondary in any way. For example, in the above primer-probe composition, the first primer and the second primer of the primer sets are structurally interchangeable. For example, the first primer contains primer signal detection regions (h) and target sequence binding regions, and the second primer contains target sequence binding regions; or for example, the first primer contains primer signal detection regions (h) and target sequence binding regions, and the second primer contains probe anchoring regions (A) and target sequence binding regions. In certain embodiments, "first primer" is also known as "forward primer", and "second primer" is also known as "reverse primer".

In the present invention, the probe is a freely designed sequence that is not paired with and does not bind to any target nucleic acids, and the probe is modified with detection labels. Preferably, the detection labels include a first detection group and a second detection group, and the first detection group and the second detection group generate a change in signal through a change in distance; preferably, the first detection group and the second detection group are spaced by 3-250 angstroms; preferably, by 3-201 angstroms; more preferably, 3-140 angstroms; and/or the first detection group is a reporter dye, and the second detection group is a quencher or other modification groups that can generate a signal change with the first detection group through fluorescence resonance energy transfer.

In the present invention, locations of the first detection group and the second detection group on the probe make the reverse complementary sequence (h') of the single-stranded pre-product specifically bind to the probe (P) and extend by >_ 0 base to form a double-stranded product. The probe may generate a detectable signal change as long as the formation of the double-stranded product can cause the locations of the first detection group and the second detection group to change. The locations of the first detection group and the second detection group are interchangeable.

In certain embodiments, in the absence of target nucleic acids to be detected, no single-stranded pre-product specifically binds to the probes, the probes are in a single-stranded state or have other secondary structures, at which point, there is a relatively short distance between the first detection group and the second detection group, and the efficiency of fluorescence resonance energy transfer is relatively high; and if the first primer contains probe anchoring regions (A), in the absence of the target nucleic acids to be detected, even if primer anchoring regions (A') of the probes are complementary with the probe anchoring regions (A) in the first primer, other parts of the probes are in a single-stranded state or have other secondary structures, at which point, there is still a relatively short distance between the first detection group and the second detection group, and the efficiency of fluorescence resonance energy transfer is relatively high. However, in the presence of the target nucleic acids to be detected, the first primer and the second primer specifically bind to target sequences respectively and extend to generate a double-stranded amplification product, with one single-stranded amplification product specifically binding to the probes to form a double-stranded product. At this point, the distance between the first detection group and the second detection group becomes long, and the efficiency of fluorescence resonance energy transfer is reduced, so that a fluorescence signal changes and may be detected by an instrument.

In certain embodiments, mixing a primer-probe composition, a sample to be detected and an amplification reagent to obtain a reaction system, and placing the reaction system in a condition that allows a nucleic acid polymerase to perform hybridization and extension reaction, so as to obtain a reaction product, specifically include the following contents.

In the condition that allows the nucleic acid polymerase to perform hybridization and extension reaction, performing PCR amplification to obtain a pre-product: a forward primer (F1) and a reverse primer (R1) specifically bind to a target sequence 1 respectively and extend to generate a double-stranded pre-amplification product, with one single-stranded pre-amplification product (S1) sequentially having a probe anchoring region (A1), a target sequence, a reverse complementary sequence (h1') of a primer signal detection region, and a reverse complementary sequence (M') of an extension blocking region from a 5' end to a 3' end; a forward primer (F2) and a reverse primer (R2) specifically bind to a target sequence 2 respectively and extend to generate a double-stranded pre-amplification product, with one single-stranded pre-amplification product (S2) sequentially having a probe anchoring region (A2), a target sequence, and a reverse complementary sequence (h2') of a primer signal detection region from a 5' end to a 3' end; optionally, if a forward primer (F3) and a reverse primer (R3) are presented, the forward primer (F3) and the reverse primer (R3) specifically bind to a target sequence 3 respectively and extend to generate a double-stranded pre-amplification product, with one single-stranded pre-amplification product (S3) sequentially having a probe anchoring region (A3), a target sequence, and a reverse complementary sequence (h3') of a primer signal detection region from a 5' end to a 3' end;
placing the above pre-product in the condition that allows the nucleic acid polymerase to perform hybridization and extension reaction (constant-temperature incubation, or a PCR amplification condition with multiple high and low temperature cycles) to obtain a reaction product: at this point, a probe signal detection region (H) of a probe (P) is reversely and complementarily paired with the reverse complementary sequence (h1') of the primer signal detection region of the single-stranded pre-amplification product (S1), and a primer anchoring region (A1') of the probe (P) is reversely and complementarily paired with the probe anchoring region (A1) at the 5' end of the single-stranded pre-amplification product (S1), so as to obtain a hybridized double-stranded product (D1) formed together with the probe (P), with a melting temperature of T1, where the reverse complementary sequence (M') of the extension blocking region at the 3' end of the single-stranded pre-amplification product (S1) is not complementary with any parts of the probe (P) or the target sequence; at this point, the probe signal detection region (H) of the probe (P) is complementarily paired with the reverse complementary sequence (h2') of the primer signal detection region at the 3' end of the single-stranded pre-amplification product (S2), a primer anchoring region (A2') of the probe (P) is reversely and complementarily paired with the probe anchoring region (A2) at the 5' end of the single-stranded pre-amplification product (S2), and the 3' end of the single-stranded pre-amplification product (S2) may continue to extend to the 5' end of the probe (P), so as to obtain part or all of reverse complementary sequences of the probe (P), i.e., completing secondary amplification with the single-stranded pre-amplification product (S2) as a primer and the probe (P) as a template, and to obtain an amplified double-stranded product (D2) formed together with the probe (P), with a melting temperature of T2, where T2 > T1; preferably, if the forward primer (F3) and the reverse primer (R3) are presented, the probe signal detection region (H) of the probe (P) is complementarily paired with the reverse complementary sequence (h3') of the primer signal detection region at the 3' end of the single-stranded pre-amplification product (S3), a primer anchoring region (A3') of the probe (P) is reversely and complementarily paired with the probe anchoring region (A3) at the 5' end of the single-stranded pre-amplification product (S3), and the 3' end of the single-stranded pre-amplification product (S3) may continue to extend to the 5' end of the probe (P), so as to obtain part or all of reverse complementary sequences of the probe (P), i.e., completing secondary amplification with the single-stranded pre-amplification product (S3) as the primer and the probe (P) as the template, and to obtain an amplified double-stranded product (D3) formed together with the probe (P), with a melting temperature of T3, where T3 > T2.

In certain embodiments, placing the reaction product at n different signal acquisition temperatures for n signal acquisitions, and analyzing whether there is a difference of presence or absence between signals obtained from two signal acquisitions at adjacent signal acquisitions temperature under a same signal channel to determine presence or absence of the target nucleic acids in the sample to be detected, specifically include the following contents.

A first signal acquisition is performed at a first signal acquisition temperature (t1, t1 ≤ T1), since the single-stranded amplification products S1, S2 and S3 form all or part of double-stranded structures D1, D2 and D3 together with the probe (P) respectively, compared to the state of the probe (P) before PCR, the distance between a first detection group and a second detection group becomes long, and the efficiency of fluorescence resonance energy transfer is relatively low, so that a fluorescence signal changes and may be detected by an instrument; heating up is performed to a second signal acquisition temperature (t2, T1 < t2 ≤ T2) for a second signal acquisition, since the second signal acquisition temperature (t2) is greater than the melting temperature of the amplified double-stranded product (D1), the amplified double-stranded product (D1) formed by the single-stranded pre-amplification product (S1) and the probe (P) cannot form the double-stranded structure, with no fluorescence signal generated, while the amplified double-stranded product (D2) formed by the single-stranded pre-amplification product (S2) and the probe (P) and the amplified double-stranded product (D3) formed by the single-stranded pre-amplification product (S3) and the probe (P) remains in a double-stranded state, with the fluorescence signal being detectable by the instrument; optionally, if the forward primer (F3) and the reverse primer (R3) are presented, heating up is performed to a third signal acquisition temperature (t3, T2 < t3 ≤ T3) for a third signal acquisition, since the third signal acquisition temperature (t3) is greater than the melting temperature of the amplified double-stranded product (D2), the amplified double-stranded product (D2) formed by the single-stranded pre-amplification product (S2) and the probe (P) cannot form the double-stranded structure, with no fluorescence signal generated, while the amplified double-stranded product (D3) formed by the single-stranded pre-amplification product (S3) and the probe (P) remains in the double-stranded state, with the fluorescence signal being detectable by the instrument.

In certain embodiments, placing the reaction product at n different signal acquisition temperatures for n signal acquisitions, and analyzing whether there is a difference of presence or absence between signals obtained from two signal acquisitions at adjacent signal acquisitions temperature under a same signal channel to determine presence or absence of the target nucleic acids in the sample to be detected, specifically include the following contents.

Optionally, if the forward primer (F3) and the reverse primer (R3) are presented, a first signal acquisition is performed at a first signal acquisition temperature (t3, T2 < t3 ≤ T3), since the first signal acquisition temperature (t3) is greater than melting temperatures of the amplified double-stranded products (D1 and D2), the amplified double-stranded product (D 1) formed by the single-stranded pre-amplification product (S 1) and the probe (P) and the amplified double-stranded product (D2) formed by the single-stranded pre-amplification product (S2) and the probe (P) cannot form the double-stranded structures, while as for the amplified double-stranded product (D3) formed by the single-stranded pre-amplification product (S3) and the probe (P), compared to the state of the probe (P) before PCR, the distance between the first detection group and the second detection group becomes long, and the efficiency of fluorescence resonance energy transfer is relatively low, so that the fluorescence signal changes and may be detected by the instrument; cooling down is performed to a second signal acquisition temperature (t2, T1 < t2 ≤ T2) for a second signal acquisition, since the second signal acquisition temperature (t2) is greater than the melting temperature of the amplified double-stranded product (D1), the amplified double-stranded product (D1) formed by the single-stranded pre-amplification product (S1) and the probe (P) cannot form the double-stranded structure, with no fluorescence signal generated, while as for the he amplified double-stranded product (D2) formed by the single-stranded pre-amplification product (S2) and the probe (P), compared to the state of the probe (P) before PCR, the distance between the first detection group and the second detection group becomes long, and the efficiency of fluorescence resonance energy transfer is relatively low, so that the fluorescence signal changes and may be detected by the instrument, and the amplified double-stranded product (D3) formed by the single-stranded pre-amplification product (S3) and the probe (P) remains in the double-stranded state, with the fluorescence signal being detectable by the instrument; cooling down is performed to a third signal acquisition temperature (t1, t1 ≤ T1) for a third signal acquisition, as for the amplified double-stranded product (D1) formed by the single-stranded pre-amplification product (S1) and the probe (P), compared to the state of the probe (P) before PCR, the distance between the first detection group and the second detection group becomes long, and the efficiency of fluorescence resonance energy transfer is relatively low, so that the fluorescence signal changes and may be detected by the instrument, and the amplified double-stranded product (D3) formed by the single-stranded pre-amplification product (S3) and the probe (P) and the amplified double-stranded product (D2) formed by the single-stranded pre-amplification product (S2) and the probe (P) remain in the double-stranded state, with the fluorescence signal being detectable by the instrument.

A second aspect of the present invention provides a device for detecting multiple target nucleic acids, including:
a reaction liquid containing part, configured to contain a plurality of micro liquids, each micro liquid containing a reaction reagent, and part of the micro liquids further containing one of a first material to be detected or a second material to be detected;
a temperature adjustment part, configured to adjust a temperature of the micro liquids in the reaction liquid containing part;
a signal detection part, configured to detect a signal generated by the micro liquids in the reaction liquid containing part;
a control part, the control part controlling the temperature adjustment part to adjust the temperature of the micro liquids in the reaction liquid containing part in such a way that the plurality of micro liquids containing the first material to be detected or the second material to be detected simultaneously generate the signal;
the control part controlling the temperature adjustment part to adjust the temperature of the micro liquids to be 11, a plurality of micro liquids containing the first material to be detected and a plurality of micro liquids containing the second material to be detected generate signals to form a first mixed signal;
the control part controlling the temperature adjustment part to adjust the temperature of the micro liquids to be t2, the plurality of micro liquids only containing the second material to be detected generate a signal to form a second signal;
wherein, t1 < t2; the control part controlling the signal detection part to acquire, at the temperatures t1 and t2, the signals generated by the micro liquids and output the first mixed signal and the second signal; and
a signal analysis part, the signal analysis part working out a first signal according to the first mixed signal and the second signal acquired by the signal detection part.

In certain embodiments, the control part controls the temperature adjustment part to adjust the temperature of the micro liquids in the reaction liquid containing part to be t3, the plurality of micro liquids containing the first material to be detected, the plurality of micro liquids containing the second material to be detected and a plurality of micro liquids containing a third material to be detected generate signals to form a second mixed signal, wherein, t3 < t1, and t3 and t1 differ by 4°C or above.

In certain embodiments, t1 and t2 differ by 4°C or above.

In certain embodiments, t1 and t2 differ by 4°C.

In certain embodiments, the signal analysis part subtracts the second signal from the first mixed signal to obtain the first signal.

In certain embodiments, when the first mixed signal and the second signal are acquired, the plurality of micro liquids are flatly laid at the bottom of the reaction liquid containing part and always maintain the same position state.

In certain embodiments, the first signal is a fluorescence signal generated from the micro liquids containing the first material to be detected, the second signal is a fluorescence signal generated from the micro liquids containing the second material to be detected, and the first mixed signal is a fluorescence signal generated from the micro liquids containing the first material to be detected and the micro liquids containing the second material to be detected.

In certain embodiments, the signal analysis part subtracts the fluorescence signal generated from the micro liquids containing the second material to be detected at the same position from the first mixed signal generated from the micro liquids to obtain the first signal.

In certain embodiments, the signal analysis part subtracts the first mixed signal generated from the micro liquids containing the first material to be detected and the micro liquids containing the second material to be detected at the same position from the second mixed signal generated from the micro liquids to obtain a third signal, and the third signal is a fluorescence signal generated from micro liquids containing the third material to be detected. In certain embodiments, each micro liquid contains a reaction reagent, which includes an amplification substance for amplifying nucleic acids and a marking substance for marking the nucleic acids. The amplification substance may amplify different nucleic acid molecules, and the marking substance can bind to the nucleic acids at a certain temperature to generate a detectable signal.

In certain embodiments, the reaction reagent includes a primer-probe composition and an amplification reagent; the amplification reagent includes a nucleic acid polymerase and dNTPs; and the primer-probe composition includes a first probe and a first primer mixture;
the first primer mixture includes at least two kinds of primer sets, and the different kinds of primer sets specifically bind to different kinds of target nucleic acids respectively;
the primer sets in the first primer mixture specifically bind to the corresponding target nucleic acids to generate a pre-product, the pre-product contains a single-stranded pre-product specifically binding to the first probe, and the single-stranded pre-product specifically binds to the first probe and extends by >_ 0 base to form a double-stranded product, and formation of the double-stranded product causes a detectable signal change.

In certain embodiments, the different kinds of primer sets in the first primer mixture and the corresponding target nucleic acids generate different single-stranded pre-products, the different single-stranded pre-products and the first probe form different double-stranded products, and the different double-stranded products have different melting temperatures; in certain embodiments, the melting temperatures of the different double-stranded products differ by 4°C or above.

In certain embodiments, the primer-probe composition further includes a second probe and a second primer mixture;
the second probe and the first probe have different base sequences and are modified with different detection labels; the second primer mixture includes at least one kind of primer set, and different kinds of primer sets specifically bind to different kinds of target nucleic acids respectively;

In certain embodiments, the primer sets in the second primer mixture specifically bind to the corresponding target nucleic acids to generate a pre-product, the pre-product contains a single-stranded pre-product specifically binding to the second probe, and the single-stranded pre-product specifically binds to the second probe and extends by >_ 0 base to form a double-stranded product, and formation of the double-stranded product causes a detectable signal change;
in certain embodiments, the different kinds of primer sets in the second primer mixture and the corresponding target nucleic acids generate different single-stranded pre-products, the different single-stranded pre-products and the second probe form different double-stranded products, and the different double-stranded products have different melting temperatures; preferably, the melting temperatures of the different double-stranded products differ by 4°C or above.

In certain embodiments, the first probe or the second probe is a sequence that does not specifically bind to any target nucleic acids, and includes a probe signal detection region (H), and sequences of the probe signal detection regions (H) of the different probes are different from each other;
each primer set includes a first primer and a second primer, and the first primer contains target sequence binding regions 1; the second primer contains primer signal detection regions (h) and target sequence binding regions 2, and the primer signal detection regions (h) are located at 5' ends of the target sequence binding regions 2; each primer signal detection region (h) is a sequence that does not specifically bind to any target nucleic acids and partially or wholly identical to the probe signal detection region (H) of the corresponding probe; sequences of the primer signal detection regions (h) of the second primers in the different primer sets are different from each other;
preferably, the first probe or the second probe further contains a primer anchoring region (A'); the first primer of the at least one kind of primer set in the first primer mixture or the second primer mixture further includes a probe anchoring region (A), and the probe anchoring region (A) is located at a 5' end of the target sequence binding region 1; the probe anchoring region (A) does not specifically bind to any target nucleic acids but specifically binds to the primer anchoring region (A');
preferably, in the first primer mixture or the second primer mixture, the second primer of at most one kind of primer set further includes an extension blocking region (M), the extension blocking region (M) is located at a 5' end of the primer signal detection region (h), and neither the extension blocking region (M) nor a complementary sequence thereof specifically binds to any probes or any target nucleic acids.

### Beneficial effects

Compared with the prior art, the primer-probe composition and the digital PCR detection method according to the present invention have the following advantages.
(1) simple method: the method according to the present invention may realize octuple reaction on digital PCR, and different targets may be distinguished by distinguishing the presence or absence of fluorescence through a finite number of photographs, which has low fluorescence recognition requirements for digital PCR, is easy to operate and saves time;
(2) multiplex detection: the method according to the present invention may simultaneously perform analysis in single-tube reaction in two dimensions of a fluorescence channel and a melting temperature. That is, by using the same fluorescence channel, different targets may be detected through different signal acquisition temperatures; or by using different fluorescence channels, the detection of the classes of the targets which are a product of the number of the fluorescence channels and signal acquisition temperature characteristics can be achieved;
(3) low fluorescence background: only by using one kind of probe for each fluorescence channel, distinguishing may be performed through different melting temperatures of amplification products, which greatly reduces a fluorescence background in PCR reaction and improves the sensitivity of detection;
(4) adjustable melting temperature: the method according to the present invention performs distinguishing by using different melting temperatures of secondary amplification products, and therefore, by adjusting the length or sequence of the primer signal detection region (h) or adjusting the location at which the primer signal detection region (h) is reversely complementary with the probe signal detection region (H) of the probe (P), a melting temperature of a secondary amplification double-stranded product formed with the probe (P) is increased or reduced;
(5) good inclusiveness: the primer probe design method according to the present invention has two parts of reverse complementary pairing with the target sequence, i.e., the forward primer (F) and the reverse primer (R), and compared with a Taqman hydrolysis probe method which requires three parts of reverse complementary pairing with the template, the primer probe design method according to the present invention has better inclusiveness and less difficulty in designing when a number of hypervariable regions, such as viral or bacterial genomes, are presented in the target sequence;
(6) low cost: the number of fluorescence probes in the reagent is reduced, which greatly reduces the reagent cost and makes large-scale promotion possible;
(7) single-tube reaction: it consumes fewer samples and thus is particularly suitable for detecting rare samples, and it may greatly increases the concentration of the samples added into detection reaction, thereby improving the sensitivity of detection. At the same time, single-tube reaction provides the possibility of subsequent extraction-free one-tube detection;
(8) simple operation: only a digital PCR instrument is required for detection, with no subsequent steps such as capillary electrophoresis or nucleic acid hybridization, at the same time, preparation is simple, and no operation is required once on the machine;
(9) not prone to pollution: according to the method according to the present invention, complete closure is realized once sample addition is completed, without the need to open a cap for subsequent detection, which greatly reduces the possibility of pollution of an experimental environment;
(10) high sensitivity: the method according to the present invention has very low requirements for the length of the target sequence, and when the target is a short-fragment nucleic acid, such as a free nucleic acid, the shorter target sequence has higher sensitivity in detection;
(11) a wide range of application: the method according to the present invention is applicable to nucleic acid detection of multiple sample types, including a serum sample, a plasma sample, a whole blood sample, a sputum sample, a swab sample, a lavage fluid sample, a fresh tissue sample, a formalin-fixed and paraffin-embedded tissue (FFPE), etc.

### Brief Description of the Drawings

Fig. 1A is a diagram of signals acquired at 50°C in Example 1; Fig. 1B is a diagram of signals acquired at 68°C in Example 1;
Fig. 2A is a diagram of signals acquired at 50°C in Example 2; Fig. 2B is a diagram of signals acquired at 68°C in Example 2; Fig. 2C is a diagram of signals acquired at 77°C in Example 2;
Fig. 3A is a diagram of signals acquired at 70°C in Example 3; Fig. 3B is a diagram of signals acquired at 50°C in Example 3;
Fig. 4A is a diagram of signals acquired at 77°C in Example 4; Fig. 4B is a diagram of signals acquired at 70°C in Example 4; and Fig. 4C is a diagram of signals acquired at 50°C in Example 4;
Fig. 5A is a schematic diagram of a device for detecting multiple target nucleic acids disclosed by an embodiment of the present application; Fig. 5B is a schematic diagram of a device for detecting multiple target nucleic acids disclosed by another embodiment of the present application; and Fig. 5C is a structural diagram of a well plate composed of a plurality of reaction liquid containing parts disclosed by yet another embodiment of the present application.

### Detailed Description of the Embodiments

The present invention will be specifically described hereinafter in conjunction with specific implementations, embodiments, and examples, and the advantages and various effects of the present invention will thus be more clearly presented. It should be understood by a person skilled in the art that, these specific implementations and embodiments are intended to illustrate the present invention and not to limit the present invention.

All of the following examples use a D600 full-automatic digital PCR analysis system and reagent consumables of Maccura Biotechnology Co., Ltd for detection and data analysis. The digital PCR analysis system disperses a reaction system containing a sample, a primer-probe composition and an amplification reagent into four reaction wells for amplification and detection (each reaction well containing a plurality of small droplets, each of which being a reaction unit). To avoid repetition, the accompanying figures of the following examples are each provided with a graphical result of the droplets in one of the reaction wells.

### Example 1

(1) A dual detection primer probe system for detecting carbapenem antibiotics resistant VIM gene mutation and carbapenem antibiotics resistant KPC gene mutation is shown in Table 1.

**Table 1 base sequences of primer probes in Example 1**

| Names of primer probes | Serial numbers | Nucleotide sequences (5'-3') | Modification |
|---|---|---|---|
| P1 | SEQ ID NO: 1 | TCGACCACGTGACAGTCTCCTCGCTGAC ACTCACTGCTGATCAGGCTCCCTGTGTC GCA | T16-ROX, T31-BHQ2 3' Spacer C3 |
| F1-1 | SEQ ID NO: 2 | GTCACGTGGTCGATTTAGCAAATTGGA CTTCCCGTAACG | / |
| R1-1 | SEQ ID NO: 3 | TTTTTTCTCCTCGCTGACACTAAACCTT CTAGAGAATGCGTGGGAATCT | / |
| F1-2 | SEQ ID NO: 4 | GTCACGTGGTCGATTTAAACTGACACTG GGCTCTGCA | / |
| R1-2 | SEQ ID NO: 5 | CACTGCTGATAAAGTCATTTGCCGTGCC ATACACT | / |

In the above primer probes,
both a forward primer F1-1 (SEQ ID NO: 2) and a reverse primer R1-1 (SEQ ID NO: 3) are specific primers designed for a target sequence of carbapenem antibiotics resistant VIM gene mutation. F1-1 has an overall length of 39 bp, with first to 23rd bases at a 3' end serving as a target sequence binding region, and first to 13th bases at a 5' end being reversely complementary with first to 13th base sequences at a 5' end of a probe P1 (SEQ ID NO: 1); and the R1-1 primer has an overall length of 49 bp, with first to 25th bases at a 3' end serving as a target sequence binding region, sixth to 21st bases at a 5' end being the same as 16th to 31 st base sequences at the 5' end of the probe P1 (SEQ ID NO: 1), and first to fifth bases at the 5' end serving as an amplification blocking region.

Both a forward primer F1-2 (SEQ ID NO: 4) and a reverse primer R1-2 (SEQ ID NO: 5) are specific primers designed for a target sequence of carbapenem antibiotics resistant KPC gene mutation. F2 has an overall length of 37 bp, with first to 21st bases at a 3' end serving as a target sequence binding region, and first to 13th bases at a 5' end being reversely complementary with first to 13th base sequences at the 5' end of the probe P1 (SEQ ID NO: 1); and the R1-2 primer has an overall length of 35 bp, with first to 22nd bases at a 3' end serving as a target sequence binding region, and first to tenth bases at a 5' end being exactly the same as 32nd to 41st base sequences at the 5' end of the probe P1 (SEQ ID NO: 1).

(2) The above primer probe system is used for detection of two kinds of target nucleic acids, and a reaction system used in detection is shown in Table 2.

**Table 2 detection reaction system in Example 1**

| Reagent composition | Concentration |
|---|---|
| 2 × PCR Reaction Buffer | 1 × |
| DNA Polymerase | 2U |
| Forward primer (F1-1) | 500 nM |
| Reverse primer (R1-1) | 100 nM |
| Forward primer (F1-2) | 500 nM |
| Reverse primer (R1-2) | 100 nM |
| Probe (P1) | 400 nM |
| Nucleic acid template of carbapenem antibiotics resistant VIM gene mutation | 2000 copies |
| Nucleic acid template of carbapenem antibiotics resistant KPC gene mutation | 1000 copies |
| Ultrapure water | Added to 20 µL |

| | |
|---|---|
| Note: 2 × PCR Reaction Buffer includes 3 mM MgCl₂, 30 mM Tris-HCl with pH of 8.3, 0.5 mM dNTP, and 70 mM (NH₄)₂SO₄. | |

(3) The specific operation steps during detection were as follows.

Sample preparation: a nucleic acid template of carbapenem antibiotics resistant VIM gene mutation and a nucleic acid template of carbapenem antibiotics resistant KPC gene mutation were simultaneously used as positive samples for detection. Pure water was used as a no template control (NTC).

Reaction preparation: after sample preparation was completed, a reaction system was configured according to the ratios in Table 2.

Digital PCR amplification and signal acquisition: after a digital PCR tube cap was sealed, the samples were gently mixed, and then briefly centrifuged and left for standing for 5 min at room temperature. A digital PCR tube was placed in a handheld centrifuge again, briefly centrifuged and transferred to a sample stand of a digital PCR instrument (D600 full-automatic digital PCR analysis system of Maccura Biotechnology Co., Ltd). Amplification and lighting procedures used were: initial denaturation at 95°C for 2 min; denaturation at 94°C for 10 s, and annealing and extending at 56°C for 30 s, for a total of 45 cycles; a first signal acquisition at 50°C; and a second signal acquisition at 68°C.

Data analysis: a result was interpreted through positive droplets at different signal acquisition temperatures.

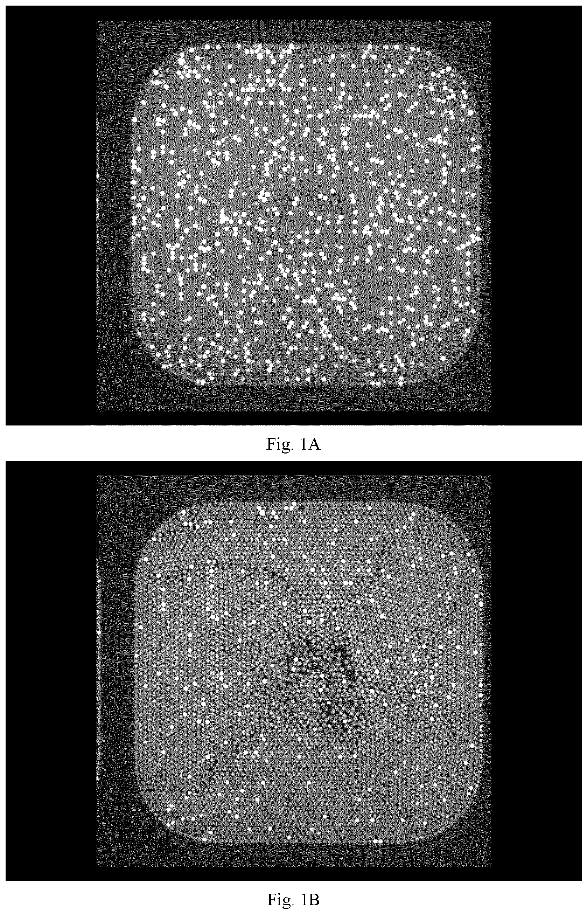

Fig. 1A is a diagram of droplets at a first signal acquisition temperature (50°C), and positive droplets (i.e., bright spots) different from a background can be seen; and Fig. 1B is a diagram of droplets at a second signal acquisition temperature (68°C), positive droplets different from the background can be seen, and the number of the positive droplets is reduced compared to Fig. 1A (part of the positive droplets in Fig. 1A is not presented at corresponding locations in Fig. 1B).

The positive droplets still presented in Fig. 1B are signals of the positive sample of carbapenem antibiotics resistant KPC gene mutation, and by analyzing the number of the positive droplets, the content of the positive sample of KPC gene mutation may further be determined; and the positive droplets not presented in Fig. 1B but presented in Fig. 1A are signals of the sample of carbapenem antibiotics resistant VIM gene mutation, and by analyzing a difference value between the positive droplets in Fig. 1A and Fig. 1B, the content of the positive sample of VIM gene mutation may further be determined.

As can be seen from Figs. 1A and 1B, by adopting the detection method of the present invention, multiple target nucleic acids can be detected. The realization of multiplex detection not only relies on fluorescence channels, but also uses different melting temperatures in the same fluorescence channel (i.e., the same signal channel), and different targets may be distinguished by distinguishing the presence or absence of fluorescence through a finite number of photographs, which has low fluorescence recognition requirements for digital PCR, is easy to operate and saves time.

### Example 2

(1) A triple detection primer probe system for detecting carbapenem antibiotics resistant VIM gene mutation, carbapenem antibiotics resistant KPC gene mutation and carbapenem antibiotics resistant OXA-48 gene mutation is shown in Table 3.

**Table 3 base sequences of primer probes in Example 2**

| Names of primer probes | Serial numbers | Nucleotide sequences (5'-3') | Modification |
|---|---|---|---|
| P2 | SEQ ID NO: 1 | TCGACCACGTGACAGTCTCCTCGCTGA CACTCACTGCTGATCAGGCTCCCTGTGT CGCA | T16-ROX, T31-BHQ2 3'Spacer C3 |
| F2-1 | SEQ ID NO: 2 | GTCACGTGGTCGATTTAGCAAATTGGA CTTCCCGTAACG | / |
| R2-1 | SEQ ID NO: 3 | TTTTTTCTCCTCGCTGACACTAAACCTT CTAGAGAATGCGTGGGAATCT | / |
| F2-2 | SEQ ID NO: 4 | GTCACGTGGTCGATTTAAACTGACACTG GGCTCTGCA | / |
| R2-2 | SEQ ID NO: 5 | CACTGCTGATAAAGTCATTTGCCGTGCC ATACACT | / |
| F2-3 | SEQ ID NO: 6 | GTCACGTGGTCGATTTATTGGCGAGGC ACGTATGA | / |
| R2-3 | SEQ ID NO: 7 | CTGTGTCGCAAAACACCGTCGAGCCAG AAACTGTCTA | / |

In the above primer probes,
both a forward primer F2-1 (SEQ ID NO: 2) and a reverse primer R2-1 (SEQ ID NO: 3) are specific primers designed for a target sequence of carbapenem antibiotics resistant VIM gene mutation. F2-1 has an overall length of 39 bp, with first to 23rd bases at a 3' end serving as a target sequence binding region, and first to 13th bases at a 5' end being reversely complementary with first to 13th base sequences at a 5' end of a probe P2 (SEQ ID NO: 1); and the R2-1 primer has an overall length of 49 bp, with first to 25th bases at a 3' end serving as a target sequence binding region, sixth to 21st bases at a 5' end being the same as 16th to 31 st base sequences at the 5' end of the probe P2 (SEQ ID NO: 1), and first to fifth bases at the 5' end serving as an amplification blocking region.

Both a forward primer F2-2 (SEQ ID NO: 4) and a reverse primer R2-2 (SEQ ID NO: 5) are specific primers designed for a target sequence of carbapenem antibiotics resistant KPC gene mutation. F2-2 has an overall length of 37 bp, with first to 21st bases at a 3' end serving as a target sequence binding region, and first to 13th bases at a 5' end being reversely complementary with first to 13th base sequences at the 5' end of the probe P2 (SEQ ID NO: 1); and the R2-2 primer has an overall length of 35 bp, with first to 22nd bases at a 3' end serving as a target sequence binding region, and first to tenth bases at a 5' end being exactly the same as 32nd to 41st base sequences at the 5' end of the probe P2 (SEQ ID NO: 1).

Both a forward primer F2-3 (SEQ ID NO: 6) and a reverse primer R2-3 (SEQ ID NO: 7) are specific primers designed for a target sequence of carbapenem antibiotics resistant OXA-48 gene mutation. F2-3 has an overall length of 35 bp, with first to 19th bases at a 3' end serving as a target sequence binding region, and first to 13th bases at a 5' end being reversely complementary with first to 13th base sequences at the 5' end of the probe P2 (SEQ ID NO: 1); and the R2-2 primer has an overall length of 37 bp, with first to 24th bases at a 3' end serving as a target sequence binding region, and first to tenth bases at a 5' end being exactly the same as first to tenth base sequences at a 3' end of the probe P2 (SEQ ID NO: 1).

(2) The above primer probe system is used for detection of three kinds of target nucleic acids, and a reaction system used in detection is shown in Table 4.

**Table 4 detection reaction system in Example 4**

| Reagent component | Concentration |
|---|---|
| 2 × PCR Reaction Buffer | 1 × |
| DNA Polymerase | 2U |
| Forward primer (F2-1) | 500 nM |
| Reverse primer (R2-1) | 100 nM |
| Forward primer (F2-2) | 500 nM |
| Reverse primer (R2-2) | 100 nM |
| Forward primer (F2-3) | 500 nM |
| Reverse primer (R2-3) | 100 nM |
| Probe (P2) | 400 nM |
| Nucleic acid template of carbapenem antibiotics resistant VIM gene mutation | 2400 copies |
| Nucleic acid template of carbapenem antibiotics resistant KPC gene mutation | 80 copies |
| Nucleic acid template of carbapenem antibiotics resistant OXA-48 gene mutation | 800 copies |
| Ultrapure water | Added to 20 µL |

| | |
|---|---|
| Note: 2 × PCR Reaction Buffer includes 3 mM MgCl₂, 30 mM Tris-HCl with pH of 8.3, 0.5 mM dNTP, and 70 mM (NH₄)₂SO₄. | |

(3) The specific operation steps during detection were as follows.

Sample preparation: a nucleic acid template of carbapenem antibiotics resistant VIM gene mutation, a nucleic acid template of carbapenem antibiotics resistant KPC gene mutation and a nucleic acid template of carbapenem antibiotics resistant OXA-48 gene mutation were simultaneously used as positive samples for detection. Pure water was used as a no template control (NTC).

Reaction preparation: after sample preparation was completed, a reaction system was configured according to the ratios in Table 4.

Digital PCR amplification and signal acquisition: after a digital PCR tube cap was sealed, the samples were gently mixed, and then briefly centrifuged and left for standing for 5 min at room temperature. A digital PCR tube was placed in a handheld centrifuge again, briefly centrifuged and transferred to a sample stand of a digital PCR instrument (D600 full-automatic digital PCR analysis system of Maccura Biotechnology Co., Ltd). Amplification and lighting procedures used were: initial denaturation at 95°C for 2 min; denaturation at 94°C for 10 s, and annealing and extending at 56°C for 30 s, for a total of 45 cycles; incubation at a constant temperature of 40°C for 3 min; a first signal acquisition at 50°C; a second signal acquisition at 68°C; and a third signal acquisition at 77°C.

Data analysis: a result was interpreted through positive droplets at different signal acquisition temperatures.

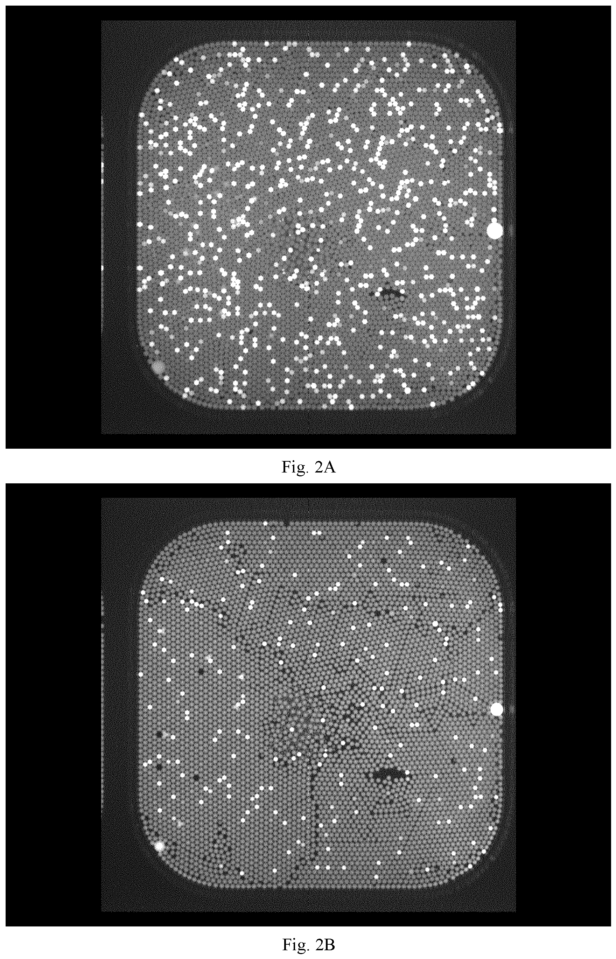

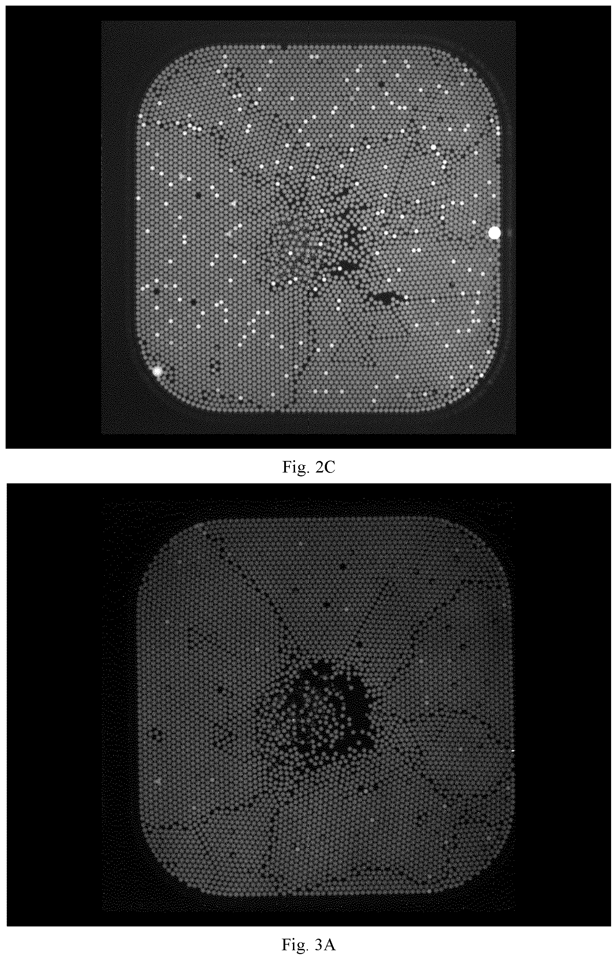

Fig. 2A is a diagram of droplets at a first signal acquisition temperature (50°C), and positive droplets (i.e., bright spots) different from a background can be seen; Fig. 2B is a diagram of droplets at a second signal acquisition temperature (68°C), positive droplets different from the background can be seen, and the number of the positive droplets is reduced compared to Fig. 2A (part of the positive droplets in Fig. 2A is not presented at corresponding locations in Fig. 2B); and Fig. 2C is a diagram of droplets at a third signal acquisition temperature (77°C), and positive droplets different from the background can be seen, and the number of the positive droplets is reduced compared to Fig. 2B (part of the positive droplets in Fig. 2B is not presented at corresponding locations in Fig. 2C).

The positive droplets still presented in Fig. 2C are signals of the positive sample of carbapenem antibiotics resistant OXA-48 gene mutation, and by analyzing the number of the positive droplets, the content of the positive sample of OXA-48 gene mutation may further be determined; the positive droplets not presented in Fig. 2C but presented in Fig. 2B are signals of carbapenem antibiotics resistant KPC gene mutation, and by analyzing a difference value between the positive droplets in Fig. 2B and Fig. 2C, the content of the positive sample of KPC gene mutation may further be determined; and the positive droplets not presented in Fig. 2B but presented in Fig. 2A are signals of the sample of carbapenem antibiotics resistant VIM gene mutation, and by analyzing a difference value between the positive droplets in Fig. 2A and Fig. 2B, the content of the positive sample of VIM gene mutation may further be determined.

As can be seen from Figs. 2A to 2C, by adopting the detection method of the present invention, multiple target nucleic acids can be detected. The realization of multiplex detection not only relies on fluorescence channels, but also uses different melting temperatures in the same fluorescence channel (i.e., the same signal channel), and different targets may be distinguished by distinguishing the presence or absence of fluorescence through a finite number of photographs, which has low fluorescence recognition requirements for digital PCR, is easy to operate and saves time.

### Example 3

(1) A dual detection primer probe system for detecting carbapenem antibiotics resistant VIM gene mutation and carbapenem antibiotics resistant OXA-48 gene mutation is shown in Table 5.

**Table 5 base sequences of primer probes in Example 3**

| Names of primer probes | Serial numbers | Nucleotide sequences (5'-3') | Modification |
|---|---|---|---|
| P3 | SEQ ID NO: 8 | CTGCTACCTCAGCTCTGAGTCAACCTG ACCTCCAATCTTCTGACTAGCGTGTGAT CGTC | T16-CY5, T31-BHQ2 3'Spacer C3 |
| F3-1 | SEQ ID NO: 9 | GCTGAGGTAGCAGTTTAGCAAA TTGGA CTTCCCGTAACG | / |
| R3-1 | SEQ ID NO: 10 | TTTTTTGAGTCAACCTGACCTAAACCTT CTAGAGAATGCGTGGGAATCT | / |
| F3-2 | SEQ ID NO: 11 | GCTGAGGTAGCAGTTTATTGGCGAGGC ACGTATGA | / |
| R3-2 | SEQ ID NO: 12 | CCAATCTTCTGAAACACCGTCGAGCCA GAAACTGTCTA | / |

In the above primer probes,
both a forward primer F3-1 (SEQ ID NO: 9) and a reverse primer R3-1 (SEQ ID NO: 10) are specific primers designed for a target sequence of carbapenem antibiotics resistant VIM gene mutation. F3-1 has an overall length of 39 bp, with first to 23rd bases at a 3' end serving as a target sequence binding region, and first to 13th bases at a 5' end being reversely complementary with first to 13th base sequences at a 5' end of a probe P3 (SEQ ID NO: 8); and the R3-1 primer has an overall length of 49 bp, with first to 25th bases at a 3' end serving as a target sequence binding region, sixth to 21st bases at a 5' end being the same as 16th to 31 st base sequences at the 5' end of the probe P3 (SEQ ID NO: 8), and first to fifth bases at the 5' end serving as an amplification blocking region.

Both a forward primer F3-2 (SEQ ID NO: 11) and a reverse primer R3-2 (SEQ ID NO: 12) are specific primers designed for a target sequence of carbapenem antibiotics resistant OXA-48 gene mutation. F3-2 has an overall length of 35 bp, with first to 19th bases at a 3' end serving as a target sequence binding region, and first to 13th bases at a 5' end being reversely complementary with first to 13th base sequences at the 5' end of the probe P3 (SEQ ID NO: 8); and the R3-2 primer has an overall length of 38 bp, with first to 24th bases at a 3' end serving as a target sequence binding region, and first to 11th bases at a 5' end being exactly the same as 32nd to 42nd base sequences at the 5' end of the probe P3 (SEQ ID NO: 8).

(2) The above primer probe system is used for detection of two kinds of target nucleic acids, and a reaction system used in detection is shown in Table 6.

**Table 6 detection reaction system in Example 3**

| Reagent composition | Concentration |
|---|---|
| 2 × PCR Reaction Buffer | 1 × |
| DNA Polymerase | 2U |
| Forward primer (F3-1) | 500 nM |
| Reverse primer (R3-1) | 100 nM |
| Forward primer (F3-2) | 500 nM |
| Reverse primer (R3-2) | 100 nM |
| Probe (P3) | 400 nM |
| Nucleic acid template of carbapenem antibiotics resistant VIM gene mutation | 10 copies |
| Nucleic acid template of carbapenem | 100 copies |
| antibiotics resistant OXA-48 gene mutation | |
| Ultrapure water | Added to 20 µL |

| | |
|---|---|
| Note: 2 × PCR Reaction Buffer includes 3 mM MgCl₂, 30 mM Tris-HCl with pH of 8.3, 0.5 mM dNTP, and 70 mM (NH₄)₂SO₄. | |

(3) The specific operation steps during detection were as follows.

Sample preparation: a nucleic acid template of carbapenem antibiotics resistant VIM gene mutation and a nucleic acid template of carbapenem antibiotics resistant OXA-48 gene mutation were simultaneously used as positive samples for detection. Pure water was used as a no template control (NTC).

Reaction preparation: after sample preparation was completed, a reaction system was configured according to the ratios in Table 6.

Digital PCR amplification and signal acquisition: after a digital PCR tube cap was sealed, the samples were gently mixed, and then briefly centrifuged and left for standing for 5 min at room temperature. A digital PCR tube was placed in a handheld centrifuge again, briefly centrifuged and transferred to a sample stand of a digital PCR instrument (D600 full-automatic digital PCR analysis system of Maccura Biotechnology Co., Ltd). Amplification and lighting procedures used were: initial denaturation at 95°C for 2 min; denaturation at 94°C for 10 s, and annealing and extending at 56°C for 30 s, for a total of 45 cycles; a constant temperature of 52°C for 3 min; a first signal acquisition at 70°C; and a second signal acquisition at 50°C.

Data analysis: a result was interpreted through positive droplets at different signal acquisition temperatures.

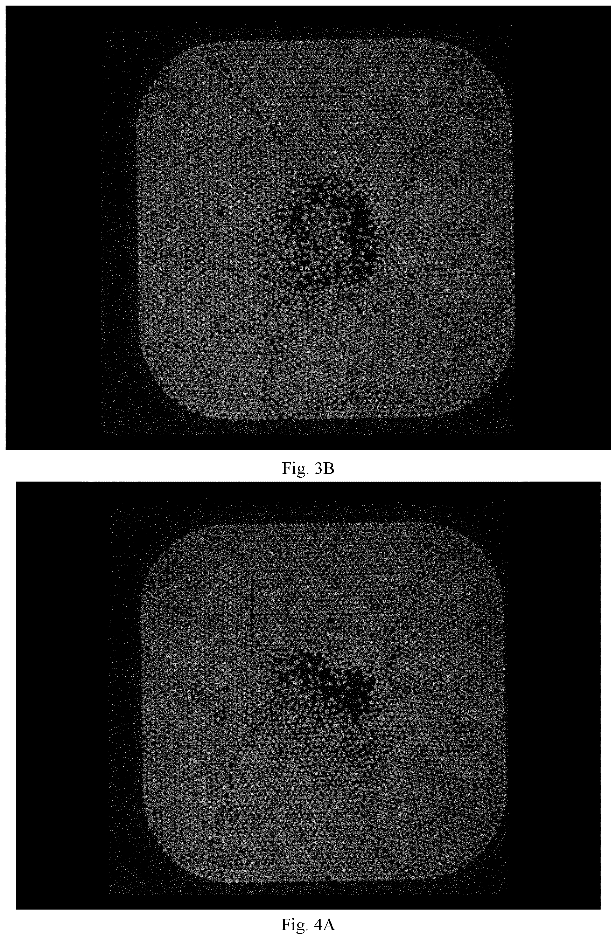

Fig. 3A is a diagram of droplets at a first signal acquisition temperature (70°C), and positive droplets (i.e., bright spots) different from a background can be seen; and Fig. 3B is a diagram of droplets at a second signal acquisition temperature (50°C), positive droplets different from the background can be seen, and the number of the positive droplets is increased compared to Fig. 3A (part of negative droplets in Fig. 3A is positive droplets at corresponding locations in Fig. 3B).

The positive droplets presented in Fig. 3A are signals of the positive sample of carbapenem antibiotics resistant OXA-48 gene mutation, and by analyzing the number of the positive droplets, the content of the positive sample of OXA-48 gene mutation may further be determined; and the positive droplets not presented in Fig. 3A but presented in Fig. 3B are signals of the sample of carbapenem antibiotics resistant VIM gene mutation, and by analyzing a difference value between the positive droplets in Fig. 3A and Fig. 3B, the content of the positive sample of VIM gene mutation may further be determined.

As can be seen from Figs. 3A and 3B, by adopting the detection method of the present invention, multiple target nucleic acids can be detected. The realization of multiplex detection not only relies on fluorescence channels, but also uses different melting temperatures in the same fluorescence channel (i.e., the same signal channel), and different targets may be distinguished by distinguishing the presence or absence of fluorescence through a finite number of photographs, which has low fluorescence recognition requirements for digital PCR, is easy to operate and saves time.

### Example 4

(1) A triple detection primer probe system for detecting carbapenem antibiotics resistant VIM gene mutation, carbapenem antibiotics resistant OXA-48 gene mutation and carbapenem antibiotics resistant IMP gene mutation is shown in Table 7.

**Table 7 base sequences of primer probes in Example 4**

| Names of primer probes | Serial numbers | Nucleotide sequences (5'-3') | Modification |
|---|---|---|---|
| P4 | SEQ ID NO: 8 | CTGCTACCTCAGCTCTGAGTCAACCTG ACCTCCAATCTTCTGACTAGCGTGTGAT CGTC | T16-CY5, T31-BHQ2 3'Spacer C3 |
| F4-1 | SEQ ID NO: 9 | GCTGAGGTAGCAGTTTAGCAAA TTGGA CTTCCCGTAACG | / |
| R4-1 | SEQ ID NO: 10 | TTTTTTGAGTCAACCTGACCTAAACCTT CTAGAGAATGCGTGGGAATCT | / |
| F4-2 | SEQ ID NO: 11 | GCTGAGGTAGCAGTTTATTGGCGAGGC ACGTATGA | / |
| R4-2 | SEQ ID NO: 12 | CCAATCTTCTGAAACACCGTCGAGCCA GAAACTGTCTA | / |
| F4-3 | SEQ ID | GCTGAGGTAGCAGTTTTAGTAGTTTGGC | |
| | NO: 13 | TGCCTGAAAGGA | |
| R4-3 | SEQ ID NO: 14 | GTGATCGTCAAAGTCACCCAAATTACCT AGACCGT | |

Both a forward primer F4-1 (SEQ ID NO: 9) and a reverse primer R4-1 (SEQ ID NO: 10) are specific primers designed for a target sequence of carbapenem antibiotics resistant VIM gene mutation. F4-1 has an overall length of 39 bp, with first to 23rd bases at a 3' end serving as a target sequence binding region, and first to 13th bases at a 5' end being reversely complementary with first to 13th base sequences at a 5' end of a probe P4 (SEQ ID NO: 18); and the R4-1 primer has an overall length of 49 bp, with first to 25th bases at a 3' end serving as a target sequence binding region, sixth to 21st bases at a 5' end being the same as 16th to 31 st base sequences at the 5' end of the probe P4 (SEQ ID NO: 18), and first to fifth bases at the 5' end serving as an amplification blocking region.

Both a forward primer F4-2 (SEQ ID NO: 11) and a reverse primer R4-2 (SEQ ID NO: 12) are specific primers designed for a target sequence of carbapenem antibiotics resistant OXA-48 gene mutation. F4-2 has an overall length of 35 bp, with first to 19th bases at a 3' end serving as a target sequence binding region, and first to 13th bases at a 5' end being reversely complementary with first to 13th base sequences at the 5' end of the probe P4 (SEQ ID NO: 8); and the R4-2 primer has an overall length of 38 bp, with first to 24th bases at a 3' end serving as a target sequence binding region, and first to 11th bases at a 5' end being exactly the same as 32nd to 42nd base sequences at the 5' end of the probe P4 (SEQ ID NO: 8).

Both a forward primer F4-3 (SEQ ID NO: 13) and a reverse primer R4-3 (SEQ ID NO: 14) are specific primers designed for a target sequence of carbapenem antibiotics resistant IMP gene mutation. F4-3 has an overall length of 40 bp, with first to 24th bases at a 3' end serving as a target sequence binding region, and first to 13th bases at a 5' end being reversely complementary with first to 13th base sequences at the 5' end of the probe P4 (SEQ ID NO: 8); and the R4-3 primer has an overall length of 35 bp, with first to 23rd bases at a 3' end serving as a target sequence binding region, and first to ninth bases at a 5' end being exactly the same as first to ninth base sequences at a 3' end of the probe P4 (SEQ ID NO: 8).

(2) The above primer probe system is used for detection of three kinds of target nucleic acids, and a reaction system used in detection is shown in Table 8.

**Table 8 detection reaction system in Example 4**

| Reagent composition | Concentration |
|---|---|
| 2 × PCR Reaction Buffer | 1 × |
| DNA Polymerase | 2U |
| Forward primer (F4-1) | 500 nM |
| Reverse primer (R4-1) | 100 nM |
| Forward primer (F4-2) | 500 nM |
| Reverse primer (R4-2) | 100 nM |
| Forward primer (F4-3) | 500 nM |
| Reverse primer (R4-3) | 100 nM |
| Probe (P4) | 400 nM |
| Nucleic acid template of carbapenem antibiotics resistant VIM gene mutation | 10 copies |
| Nucleic acid template of carbapenem antibiotics resistant OXA-48 gene mutation | 15 copies |
| Nucleic acid template of carbapenem antibiotics resistant IMP gene mutation | 100 copies |
| Ultrapure water | Added to 20 µL |

| | |
|---|---|
| Note: 2 × PCR Reaction Buffer includes 3 mM MgCl₂, 30 mM Tris-HCl with pH of 8.3, 0.5 mM dNTP, and 70 mM (NH₄)₂SO₄. | |

(3) The specific operation steps during detection were as follows.

Sample preparation: a nucleic acid template of carbapenem antibiotics resistant VIM gene mutation, a nucleic acid template of carbapenem antibiotics resistant OXA-48 gene mutation and a nucleic acid template of carbapenem antibiotics resistant IPM gene mutation were simultaneously used as positive samples for detection. Pure water was used as a no template control (NTC).

Reaction preparation: after sample preparation was completed, a reaction system was configured according to the ratios in Table 8.

Digital PCR amplification and signal acquisition: after a digital PCR tube cap was sealed, the samples were gently mixed, and then briefly centrifuged and left for standing for 5 min at room temperature. A digital PCR tube was placed in a handheld centrifuge again, briefly centrifuged and transferred to a sample stand of a digital PCR instrument (D600 full-automatic digital PCR analysis system of Maccura Biotechnology Co., Ltd). Amplification and lighting procedures used were: initial denaturation at 95°C for 2 min; denaturation at 94°C for 10 s, and annealing and extending at 56°C for 30 s, for a total of 45 cycles; incubation at a constant temperature of 40°C for 3 min; a first signal acquisition at 77°C; a second signal acquisition at 70°C; and a third signal acquisition at 50°C.

Data analysis: a result was interpreted through positive droplets at different signal acquisition temperatures.

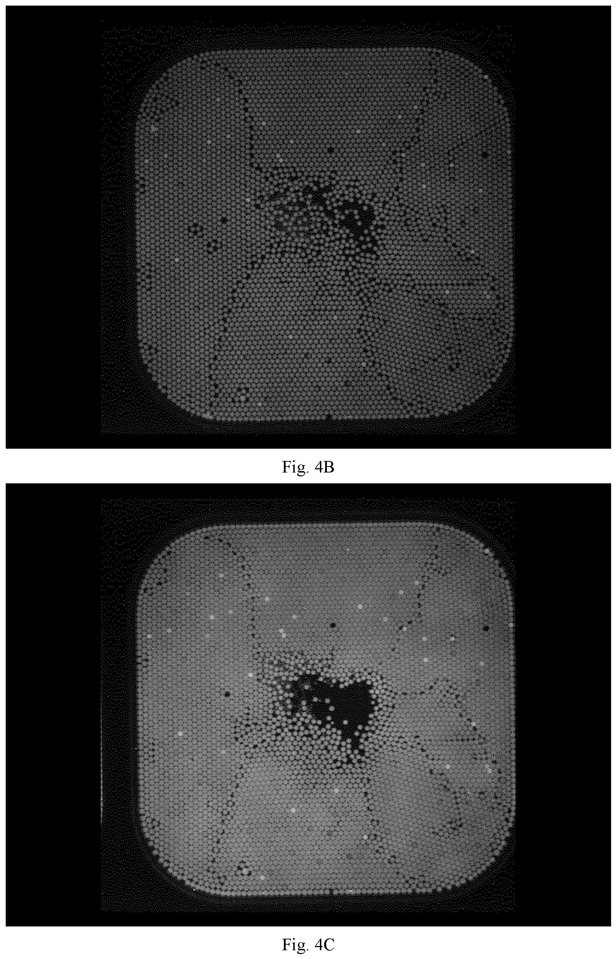

Fig. 4A is a diagram of droplets at a first signal acquisition temperature (77°C), and positive droplets (i.e., bright spots)different from a background can be seen; Fig. 4B is a diagram of droplets at a second signal acquisition temperature (70°C), positive droplets different from the background can be seen, and the number of the positive droplets is increased compared to Fig. 4A (part of negative droplets in Fig. 4A is positive droplets at corresponding locations in Fig. 4B); and Fig. 4C is a diagram of droplets at a third signal acquisition temperature (50°C), positive droplets different from the background can be seen, and the number of the positive droplets is increased compared to Fig. 4B (part of negative droplets in Fig. 4B is positive droplets at corresponding locations in Fig. 4C).

The positive droplets presented in Fig. 4A are signals of the positive sample of carbapenem antibiotics resistant IMP gene mutation, and by analyzing the number of the positive droplets, the content of the positive sample of IMP gene mutation may further be determined; the positive droplets not presented in Fig. 4A but presented in Fig. 4B are signals of the sample of carbapenem antibiotics resistant OXA-48 gene mutation, and by analyzing a difference value between the positive droplets in Fig. 4A and Fig. 4B, the content of the positive sample of OXA-48 gene mutation may further be determined; and the positive droplets not presented in Fig. 4B but presented in Fig. 4C are signals of the sample of carbapenem antibiotics resistant VIM gene mutation, and by analyzing a difference value between the positive droplets in Fig. 4B and Fig. 4C, the content of the positive sample of VIM gene mutation may further be determined.

As can be seen from Figs. 4A and 4B, by adopting the detection method of the present invention, multiple target nucleic acids can be detected. The realization of multiplex detection not only relies on fluorescence channels, but also uses different melting temperatures in the same fluorescence channel (i.e., the same signal channel), and different targets may be distinguished by distinguishing the presence or absence of fluorescence through a finite number of photographs, which has low fluorescence recognition requirements for digital PCR, is easy to operate and saves time.

### Specific implementation 2

An embodiment of the present application provides a multiple target nucleic acid detection device for multiplex detection of nucleic acids, which can distinguish different targets by distinguishing the presence or absence of fluorescence through a finite number of photographs, has low fluorescence recognition requirements for digital PCR, is easy to operate and saves time.

In order to enable a person skilled in the art to better understand the solutions of the present application, the technical solutions in the embodiments of the present application will be described below in conjunction with the drawings in the embodiments of the present application. Apparently, the described embodiments are some, rather than all of the embodiments of the present application. All the embodiments based on the present application shall fall within the scope of protection of the present application.

It should be noted that terms "including" and "having" and any variants thereof in the embodiments and drawings of the present application are intended to cover non-exclusive inclusion. For example, a process, method, system, product or apparatus including a series of steps or units is not limited to steps or units that are expressly listed, but optionally further includes steps or units that are not listed, or optionally further includes other steps or units that are inherent to these processes, methods, products or apparatuses.

It may be understood that terms "first", "second", etc. used in the present application may be used herein for describing various elements, but these elements are not limited to these terms. These terms are only used for distinguishing the first element from another element. For example, without departing from the scope of the present application, a first material to be detected may be referred to as a second material to be detected, and similarly, the second material to be detected may be referred to as the first material to be detected. Both the first material to be detected and the second material to be detected are materials to be detected, but they are not the same material to be detected. In addition, it should be noted that the term "a plurality of' used in the embodiments of the present application refers to two, or two or more.

The technical solutions of the present application will be further illustrated below by way of embodiments.

The device for detecting multiple target nucleic acids according to the implementation is used for detecting a sample to be detected. The sample to be detected may include one kind of component to be detected, and may also include two, three, four, five or even more kinds of components to be detected. The plurality kinds of components to be detected in the sample to be detected may be detected through the nucleic acid detection device according to the implementation.

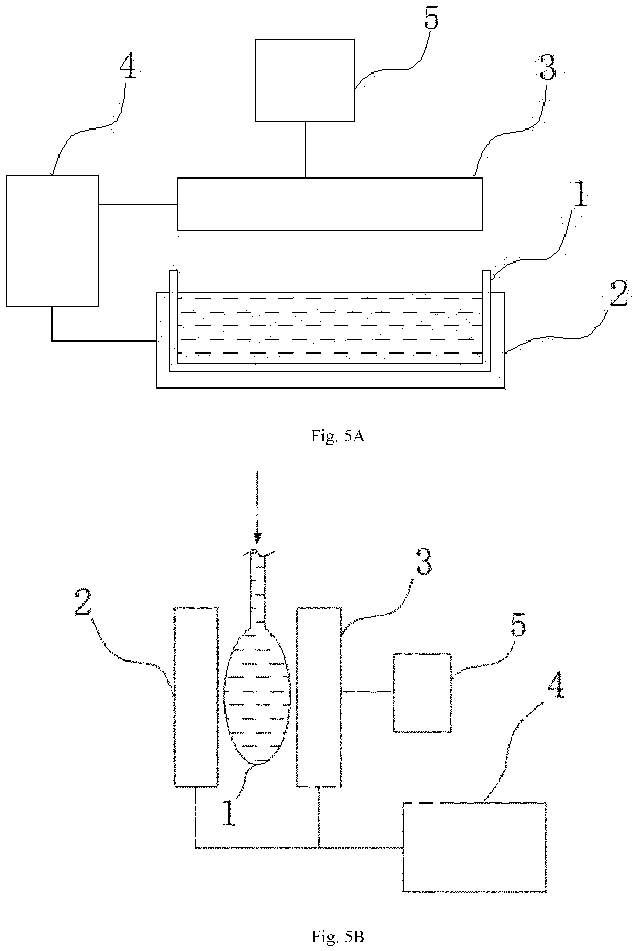

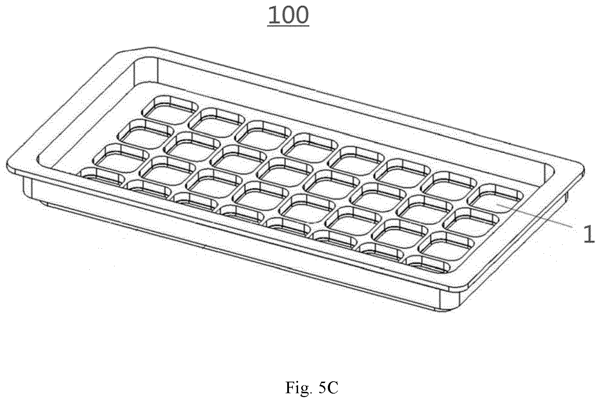

Referring to Figs. 5A and5B, the device for detecting multiple target nucleic acids according to the implementation includes a reaction liquid containing part 1, a temperature adjustment part 2, a signal detection part 3, a control part 4 and a signal analysis part 5. The reaction liquid containing part 1 is configured to contain a sample to be detected containing a component to be detected and a reaction reagent capable of reacting with the component to be detected to provide a place for various reaction between the sample to be detected and the reaction reagent. The temperature adjustment part 2 is configured to adjust the temperature of a mixed liquid in the reaction liquid containing part 1 to make the sample to be detected and the reaction reagent react differently at different temperatures. The signal detection part 3 is configured to detect a signal generated from a specific substance undergoing specific reaction in the mixed liquid, and the signal may be an optical signal and may also be an electric signal. The control part 4 is configured to control the temperature adjustment part 2 to adjust the temperature of the mixed liquid in the reaction liquid containing part 1 in different temperature modes, and control the signal detection part 3 to detect the signal generated from the specific substance in the mixed liquid. The signal analysis part 5 is configured to analyze the signal acquired by the signal detection part 3.

### I. Sample to be detected

The sample to be detected is a body fluid from a human body or an animal body, and includes different substances to be detected, which may be nucleic acids or proteins. The implementation illustrates the technical solutions with the substances to be detected being the nucleic acids. For the sample to be detected directly taken from the human body or the animal body, nucleic acid extraction purification operation may be performed to extract the nucleic acids, which may be performed manually or with a specialized nucleic acid extractor. For the sample to be detected directly taken from the human body or the animal body, the nucleic acid extraction purification operation may also not be performed. The sample to be detected includes different nucleic acid molecules to be detected, such as a first material to be detected (a first target nucleic acid to be detected), a second material to be detected (a second target nucleic acid to be detected), a third material to be detected (a third target nucleic acid to be detected), a fourth material to be detected (a fourth target nucleic acid to be detected), and a fifth material to be detected (a fifth target nucleic acid to be detected).

### II. Reaction reagent

The reaction reagent includes an amplification substance for amplifying nucleic acids and a marking substance for marking the nucleic acids. The amplification substance may amplify different nucleic acid molecules, and the marking substance can bind to the nucleic acids at a certain temperature to generate a detectable signal. The different nucleic acids bind to the marking substance over different temperature ranges, and separate from the marking substance over different temperature ranges after binding. Primers in the amplification substance for amplifying the different nucleic acids are different, and the marking substance for marking the different nucleic acids may be the same and may also be different. In certain embodiments, the same marking substance is used for marking the different nucleic acids.

In certain embodiments, the reaction reagent includes a primer-probe composition and an amplification reagent. The amplification reagent includes a nucleic acid polymerase and dNTPs. The primer-probe composition includes a probe and a primer mixture. The primer mixture includes at least two kinds of the primer sets, and the different kinds of primer sets specifically bind to the different kinds of target nucleic acids respectively; and the primer sets in the primer mixture specifically bind to the corresponding target nucleic acids to generate a pre-product, the pre-product contains a single-stranded pre-product specifically binding to a first probe, and the single-stranded pre-product specifically binds to the first probe and extends by >_ 0 base to form a double-stranded product, and the formation of the double-stranded product causes a detectable signal change. In certain embodiments, the different kinds of primer sets in a first primer mixture and the corresponding target nucleic acids generate different single-stranded pre-products, the different single-stranded pre-products and the first probe form different double-stranded products, and the different double-stranded products have different melting temperatures; and in certain embodiments, the melting temperatures of the different double-stranded products differ by 4°C or above.

### III. Micro liquids

The micro liquids may be tiny droplets formed in an emulsion, and may also be trace liquids contained in micro wells of a container. Each micro liquid contains at most one unit of nucleic acid molecule and a reaction reagent sufficient to amplify and mark a plurality kinds of target nucleic acids. For example, if there are two kinds of target nucleic acids to be detected, i.e., first materials to be detected and second materials to be detected, in the sample to be detected, after undergoing micro-partition treatment, each first material to be detected and each second material to be detected are distributed into different micro liquids, and there is a sufficient number of reagent in one micro liquid, which may not only meet the demand for amplifying and marking the first material to be detected in the micro liquid, but also meet the demand for amplifying and marking the second material to be detected in the micro liquid.

The process of making micro liquids may adopt a method in which the micro liquids are formed through high-frequency vibration: the sample and the sufficient number of reagent are sucked through a microchannel, the microchannel stretches under an oily liquid for high-frequency vibration, micro liquids of the sample and the reagent in the microchannel are discharged at a certain velocity in the vibration process, so that micro droplets, i.e., the micro liquids are thrown out through vibration, and the micro liquids are incompatible and non-reactive with the oily liquid. By setting a reasonable vibration frequency, vibration amplitude and discharge velocity of the micro liquids, it may be ensured that there is at most only one unit of nucleic acid molecule in each formed micro liquid.

It is also possible to provide a plurality of micro-partition regions in the container, so that by adding the micro liquids of the sample to be detected and the reaction reagent into the micro-partition regions, there is at most one unit of nucleic acid molecule in each micro-partition region.

### IV. Reaction liquid containing part

As shown in Fig. 5A, the reaction liquid containing part is a box-like container with an upward opening. Nucleic acids to be detected and a reagent are added into the box-like container through a liquid filling mechanism (i.e., the method in which micro liquids are formed through high-frequency vibration as described above), and the nucleic acids to be detected and the reagent may also be added into the box-like container by a laboratorian.

As shown in Fig. 5B, the reaction liquid containing part is a cavity communicating with a microchannel, and the nucleic acids to be detected and the reagent are driven into the cavity by a microfluid driving structure.

In order to realize absolute quantitative detection of target nucleic acids to be detected, micro liquids containing the nucleic acids to be detected and the reagent are encapsulated in 500 or more, e.g., about 20,000 micro liquids, each of which contains one or no target nucleic acid molecules to be detected. Regardless of the number of classes of materials to be detected in the sample, all the materials to be detected are distributed into different micro liquids (it is an ideal state, and it is possible that a small number of micro liquids each contain one or more target nucleic acid molecules to be detected).

In the embodiment, illustration is made with the reaction liquid containing part being the box-like container. An oily liquid is injected into the reaction liquid containing part, and the oily liquid is incompatible and non-reactive with the sample and the reagent. The micro liquids are formed in the oily liquid in a mode of high-frequency vibration, and flatly laid at the bottom of the reaction liquid containing part under the force of their own gravities.

As shown in Fig. 1C, a well plate 100 is composed of a plurality of reaction liquid containing parts 1, the bottoms of which are each in a flat plate shape, so that the plurality of micro liquids are flatly laid at the bottoms of the reaction liquid containing parts 1.

### V. Temperature adjustment part

As shown in Fig. 5A and Fig. 5B, the temperature adjustment part 2 is provided below, above or to the side of the reaction liquid containing part 1, which may provide heat to the reaction liquid containing part 1 and may also absorb heat from the reaction liquid containing part 1, thereby realizing the functions of heating up and cooling down of the micro liquids in the reaction liquid containing part 1. The temperature adjustment part 2 may vary the temperature of the micro liquids in the reaction liquid containing part 1 within a range of 4°C to 105°C. A shown in Fig. 5A, when the reaction liquid containing part 1 is the box-like container with an upward opening, a placement location special for placing the reaction liquid containing part may be provided on the temperature adjustment part, and the reaction liquid containing part 1 is placed on the placement location when the temperature of the reaction liquid containing part 1 needs to be adjusted. As shown in Fig. 5B, when the reaction liquid containing part 1 is the cavity communicating with the microchannel, the temperature adjustment part 2 may be provided in a reasonable orientation of the reaction liquid containing part 1 in combination with factors such as heating efficiency.

In the embodiment, the temperature adjustment part 2 is provided below the well plate 100 composed of the plurality of reaction liquid containing parts 1, and contacts to heat and cool the flat well plate 100, so that the temperature of the micro liquid in each reaction liquid containing part 1 in the well plate 100 changes with the temperature rise and fall of the well plate 100.

### VI. Signal detection part

As shown in the figure, the signal detection part 3 is provided in the vicinity of the reaction liquid containing part 1. Depending on signals generated from the micro liquids in the reaction liquid containing part 1, the signal detection part may be a device (a camera or a photoelectric device) for detecting optical signals (e.g., a fluorescence signal) or a device capable of detecting electric signals. For example, when the signal detection part is the camera, the signals flatly laid in the reaction liquid containing part may be photographed. The signal detection part 3 is movable relative to the reaction liquid containing part 1, and when it is required to detect signals, a controller controls a driving mechanism to move the signal detection part 3 to the position close to the reaction liquid containing part 1 (e.g., above, as shown in Fig. 5A). As shown in Fig. 5B, the signal detection part 3 may also be fixedly provided relative to the reaction liquid containing part.

### VII. Control part

As shown in the figure, the control part 4 is communicably connected to the temperature adjustment part 2 and the signal detection part 3. The control part may include a first control part and a second control part (not shown in the figure). The first control part is communicably connected to the temperature adjustment part to control the temperature adjustment part to run in different modes, and the second control part is communicably connected to the signal detection part to control the signal detection part to acquire the signals from the micro liquids in the reaction liquid containing part. As shown in Figs. 5A and 5B, the control part may also be a single part, which may simultaneously control the temperature adjustment part and the signal detection part.

### VIII. Signal analysis part

As shown in the figure, the signal analysis part 5 is communicably connected to the signal detection part 3. The signal analysis part is configured to perform analysis according to the signals acquired by the signal detection part to obtain signals of target nucleic acids to be detected, thereby obtaining information, such as whether a sample to be detected contains the target nucleic acids to be detected, and the types and number of the obtained target nucleic acids to be detected.

### Embodiment 1

The embodiment exemplarily describes a device and method for dual detection: a sample to be detected contains a first material to be detected (a first target nucleic acid to be detected) and a second material to be detected (a second target nucleic acid to be detected). It should be noted that a first associated product to be detected generated from the first material to be detected (the target nucleic acid to be detected undergoes operations such as polymerase chain reaction (PCR) and/or nucleic acid hybridization and/or nucleic acid enzyme digestion to generate the corresponding associated product to be detected, and under normal conditions, the associated product to be detected is a nucleic acid) may bind to a marking substance in a reagent; and a second associated product to be detected generated from the second material to be detected may also bind to the marking substance in the reagent. In the example, in a certain temperature mode, the first associated product to be detected is generated from the first material to be detected and binds to the marking substance, and at the same time, in this temperature mode, the second associated product to be detected is generated from the second material to be detected and binds to the marking substance. However, the first associated product to be detected and the second associated product to be detected have different separation temperatures. The first associated product to be detected may be separated from the marking substance in the reagent within a first separation temperature range, and the second associated product to be detected may be separated from the marking substance in the reagent within a second separation temperature range. The first separation temperature range and the second separation temperature range have different minimum temperatures. In the example, the minimum temperature of the second separation temperature range is greater than that of the first separation temperature range. For example, the first associated product to be detected may be separated from the marking substance at a temperature greater than or equal to a temperature t1, and the second associated product to be detected may be separated from the marking substance at a temperature greater than or equal to a temperature t2, where the temperature t2 is greater than the temperature t1.

In order to realize multiplex detection of different target nucleic acids to be detected in the sample to be detected, and distinguish the different targets (target nucleic acids) by distinguishing the presence or absence of fluorescence through a finite number of photographs, in the embodiment, a mixed liquid of the sample to be detected and the reaction reagent (containing a primer-probe composition and an amplification reagent) is added to the reaction liquid containing part to form a plurality of micro liquid, and the control part performs the following controls.

The control part controls the temperature adjustment part to adjust the temperature of the micro liquids in the reaction liquid containing part, so that the target nucleic acids to be detected in the sample to be detected generate associated products to be detected associated therewith (the nucleic acids to be detected contain the first nucleic acid to be detected and the second nucleic acid to be detected, but are presented in different micro liquids); and in this process, the micro liquids may undergo a plurality of temperature cycles, in each of which the micro liquids will change to a plurality of different temperatures and maintain at this temperature for a certain time, and there may be a plurality of cycles, e.g., 20-60, e.g., about 40. In each cycle, the micro liquids will reach a high-temperature state, double-stranded nucleic acids become single-stranded nucleic acids, then the micro liquids reach a low-temperature state, and the single-stranded nucleic acids undergo annealing and extending processes (annealing and extending may also be performed at different temperatures respectively, and the extending temperature is greater than the annealing temperature). After the plurality of temperature cycles, the numbers of the different target nucleic acids to be detected and/or the different associated products to be detected in the sample to be detected are increased (i.e., the quantities of the target nucleic acids to be detected and/or the associated products to be detected in each micro liquid are increased, but the types of the nucleic acids to be detected remain unchanged), which is easier to detect. By reasonably setting the annealing temperature in the amplification process, the target nucleic acids to be detected and/or the associated products to be detected are increased in number, and bind to the marking substance in the reagent, so that the plurality of micro liquids containing the first material to be detected or the second material to be detected simultaneously generate signals.

Before performing 40 temperature cycles, the micro liquids may be heated to a temperature t0, so that the nucleic acids to be detected in the sample are initially denaturated for a period of time (about 2-5 min). Initial denaturation for a period of time is designed to change the target nucleic acids from a double-stranded state into a single-stranded state.

After completing the 40 temperature cycles, the control part controls the temperature adjustment part to adjust the temperature of the micro liquids to be t1, a plurality of micro liquids containing the first material to be detected and a plurality of micro liquids containing the second material to be detected generate signals to form a first mixed signal. At the temperature t1, the second material to be detected or the associated product thereof is in a state of binding to the marking substance, thus generating a signal, and the first material to be detected or the associated product thereof is also in a state of binding to the marking substance, thus generating a signal. Since a single nucleic acid molecule to be detected is distributed into one micro liquid, that is, there is only one nucleic acid molecule to be detected in one micro liquid, this nucleic acid molecule to be detected may be the first nucleic acid to be detected and may also be the second nucleic acid to be detected, at the temperature t1, both the micro liquids containing the first nucleic acid to be detected and the micro liquids containing the second nucleic acid to be detected can generate signals. If a plurality of micro liquids overlap, signals emitted from such micro liquids are invalid signals; and signals emitted from single micro liquids that do not overlap are valid signals.

Next, the control part controls the temperature adjustment part to adjust the temperature of the micro liquids to be t2 (t2 > t1), and a plurality of micro liquids only containing the second material to be detected generate a signal to form a second signal. At the temperature t2, the first material to be detected or the associated product thereof is separated from the marking substance, thus generating no detectable signals. However, the second material to be detected or the associated product thereof still maintains in a state of binding to the marking substance, thus generating a signal. Similarly, since a single nucleic acid molecule is distributed into one micro liquid, that is, there is only one nucleic acid molecule in one micro liquid, this nucleic acid molecule to be detected may be the first nucleic acid to be detected and may also be the second nucleic acid to be detected, at the temperature t2, only the plurality of micro liquids where the second nucleic acid to be detected is located can generate signals. If a plurality of micro liquids overlap, signals emitted from such micro liquids are invalid signals; and signals emitted from single micro liquids that do not overlap are valid signals.

In order to better distinguish the first mixed signal and the second signal, t1 and t2 differ by 4°C or above, e.g., by 4-40°C, e.g., by 4-30°C, 4-20°C, 4-18°C, 4-15°C, 4-12°C, 4-10°C, 4-8°C, 4-6°C, 4-5°C, or 4°C.

In order to obtain a more accurate first signal, when the first mixed signal and the second signal are acquired, the plurality of micro liquids are flatly laid at the bottom of the reaction liquid containing part and always maintain the same position state.

When the signal detection part detects signals generated from the micro liquids through the above controls of the control part, the numbers of classes of target nucleic acids contained in signals obtained from two adjacent signal acquisitions differ by one (i.e., the first mixed signal contains the signals of the first material to be detected and the second material to be detected, but the second signal only contains the signal of the first material to be detected).

In order to obtain the types and number of the target nucleic acids in the sample to be detected, and realize multiplex detection of different nucleic acids to be detected in the sample to be detected, the signal analysis part performs the following operations.

A first signal is worked out according to the first mixed signal and the second signal acquired by the signal detection part. The signal analysis part subtracts the second signal from the first mixed signal to obtain the first signal. Specifically, the signal analysis part subtracts the fluorescence signal generated from the micro liquids containing the second material to be detected at the same position from the first mixed signal generated from the micro liquids to obtain the first signal. The first signal is a fluorescence signal generated from the micro liquids containing the first material to be detected, the second signal is a fluorescence signal generated from the micro liquids containing the second material to be detected, and the first mixed signal is a fluorescence signal generated from the micro liquids containing the first material to be detected and the micro liquids containing the second material to be detected.

According to the second signal acquired by the signal detection part, the fluorescence signal generated from the micro liquids containing the second material to be detected may be directly obtained.

Since a single nucleic acid molecule to be detected iss distributed into one micro liquid, that is, there is at most one nucleic acid molecule to be detected in one micro liquid (some micro liquids contain no nucleic acid molecules to be detected and thus generate no signals at each signal acquisition temperature), this nucleic acid molecule to be detected may be the first nucleic acid to be detected and may also be the second nucleic acid to be detected. By analyzing the number of the micro liquids generating signals in the first mixed signal, the total number of the first material to be detected and the second material to be detected is obtained; by analyzing the number of the micro liquids generating signals in the second signal, the number of the second material to be detected is obtained; and by subtracting the number of the second material to be detected generating the second signal from the total number of the first material to be detected and the second material to be detected generating the first mixed signal, the number of the first material to be detected is obtained.

### Embodiment 2

The embodiment exemplarily describes a device and method for triple detection: a sample to be detected contains a first material to be detected (a first target nucleic acid to be detected), a second material to be detected (a second target nucleic acid to be detected), and a third material to be detected (a third target nucleic acid to be detected). Similarly, a first associated product to be detected generated from the first material to be detected (the target nucleic acid to be detected undergoes operations such as polymerase chain reaction (PCR) and/or nucleic acid hybridization and/or nucleic acid enzyme digestion to generate the corresponding associated product to be detected, and under normal conditions, the associated product to be detected is a nucleic acid) may bind to a marking substance in a reagent; and a second associated product to be detected generated from the second material to be detected may bind to the marking substance in the reagent, and a third associated product to be detected generated from the third material to be detected may bind to the marking substance in the reagent. In the embodiment, in a certain temperature mode, the first associated product to be detected is generated from the first material to be detected and binds to the marking substance. At the same time, in this temperature mode, the second associated product to be detected is generated from the second material to be detected and binds to the marking substance, and the third associated product to be detected generated from the third material to be detected is generated from the second material to be detected and binds to the marking substance. However, the first associated product to be detected, the second associated product to be detected and the third associated product to be detected have different separation temperatures. The first associated product to be detected may be separated from the marking substance in the reagent within a first separation temperature range, the second associated product to be detected may be separated from the marking substance in the reagent within a second separation temperature range, and the third associated product to be detected may be separated from the marking substance in the reagent within a third separation temperature range. The first separation temperature range, the second separation temperature range and the third separation temperature range have different minimum temperatures. In the embodiment, the minimum temperature of the third separation temperature range is less than that of the first separation temperature range, and the minimum temperature of the first separation temperature range is less than that of the second separation temperature range. For example, the first associated product to be detected may be separated from the marking substance at a temperature greater than or equal to a temperature t1, the second associated product to be detected may be separated from the marking substance at a temperature greater than or equal to a temperature t2, and the third associated product to be detected may be separated from the marking substance at a temperature greater than or equal to a temperature t3, where the temperatures t3 < t1 < t2.

In order to realize triple detection of different target nucleic acids to be detected in the sample to be detected, and distinguish the different targets (target nucleic acids) by the distinguishing presence or absence of fluorescence through a finite number of photographs, in the embodiment, a mixed liquid of the sample to be detected and the reaction reagent (containing a primer-probe composition and an amplification reagent) is added to the reaction liquid containing part to form a plurality of micro liquid, and the control part performed the following controls.

The control part controls the temperature adjustment part to adjust the temperature of the micro liquids in the reaction liquid containing part, so that the target nucleic acids to be detected in the sample to be detected generate associated products to be detected associated therewith (the target nucleic acids to be detected contain the first material to be detected, the second material to be detected and the third material to be detected, but they are presented in different micro liquids); and in this process, the micro liquids may undergo a plurality of temperature cycles, in each of which the micro liquids will change to a plurality of different temperatures and maintain at this temperature for a certain time, and there may be a plurality of cycles, e.g., 20-60, e.g., about 40. In each cycle, the micro liquids will reach a high-temperature state, double-stranded nucleic acids become single-stranded nucleic acids, then the micro liquids reach a low-temperature state, and the single-stranded nucleic acids undergo annealing and extending processes (annealing and extending may also be performed at different temperatures respectively, and the extending temperature is greater than the annealing temperature). After the plurality of temperature cycles, the numbers of the different target nucleic acids to be detected and/or the different associated products to be detected in the sample to be detected are increased (i.e., the quantities of the target nucleic acids to be detected and/or the associated products to be detected in each micro liquid are increased, but the types of the nucleic acids to be detected remain unchanged), which is easier to detect. By reasonably setting the annealing temperature in the amplification process, the target nucleic acids to be detected and/or the associated products to be detected are increased in number, and bind to the marking substance in the reagent, so that the plurality of micro liquids containing the first material to be detected or the second material to be detected or the third material to be detected simultaneously generate signals.

Before performing 40 temperature cycles, the micro liquids may be heated to a temperature t0, so that the nucleic acids to be detected in the sample are initially denaturated for a period of time (about 2-5 min). Initial denaturation for a period of time is designed to change the target nucleic acids from a double-stranded state into a single-stranded state.

After completing the 40 temperature cycles, the control part controls the temperature adjustment part to adjust the temperature of the micro liquids to be t3, a plurality of micro liquids containing the first material to be detected, a plurality of micro liquids containing the second material to be detected and a plurality of micro liquids containing the third material to be detected generate signals to form a second mixed signal. At the temperature t3, the second material to be detected or the associated product thereof is in a state of binding to the marking substance, thus generating a signal, the first material to be detected or the associated product thereof is also in a state of binding to the marking substance, thus generating a signal, and the third material to be detected or the associated product thereof is also in a state of binding to the marking substance, thus generating a signal. Since a single nucleic acid molecule to be detected is distributed into one micro liquid, that is, there is only one nucleic acid molecule to be detected in one micro liquid, this nucleic acid molecule to be detected may be the first nucleic acid to be detected and may also be the second nucleic acid to be detected or the third nucleic acid to be detected, at the temperature t3, all of the micro liquids containing the first nucleic acid to be detected, the micro liquids containing the second nucleic acid to be detected and the micro liquids containing the third nucleic acid to be detected can generate signals. If a plurality of micro liquids overlapped, signals emitted from such micro liquids are invalid signals; and signals emitted from single micro liquids that do not overlap are valid signals.

Next, the control part controls the temperature adjustment part to adjust the temperature of the micro liquids to be t1 (t1 > t3), and a plurality of micro liquids containing the first material to be detected and a plurality of micro liquids containing the second material to be detected generate signals to form a first mixed signal. At the temperature t1, the second material to be detected or the associated product thereof is in a state of binding to the marking substance, thus generating a signal, and the first material to be detected or the associated product thereof is also in a state of binding to the marking substance, thus generating a signal, but the third material to be detected or the associated product thereof is in a state of being separated from the marking substance, thus generating no signals. Since a single nucleic acid molecule to be detected is distributed into one micro liquid, that is, there is only one nucleic acid molecule to be detected in one micro liquid, this nucleic acid molecule to be detected may be the first nucleic acid to be detected and may also be the second nucleic acid to be detected, at the temperature t1, both the micro liquids containing the first nucleic acid to be detected and the micro liquids containing the second nucleic acid to be detected can generate signals. If a plurality of micro liquids overlap, signals emitted from such micro liquids are invalid signals; and signals emitted from single micro liquids that do not overlap are valid signals.

Next, the control part controls the temperature adjustment part to adjust the temperature of the micro liquids to be t2 (t2 > 11), and a plurality of micro liquids only containing the second material to be detected generate a signal to form a second signal. At the temperature t2, the first material to be detected or the associated product thereof is separated from the marking substance, and the third material to be detected or the associated product thereof is separated from the marking substance, thus both generating no detectable signals. However, the second material to be detected or the associated product thereof still maintains in a state of binding to the marking substance, thus generating a signal. Similarly, since a single nucleic acid molecule is distributed into one micro liquid, that is, there is only one nucleic acid molecule in one micro liquid, only the plurality of micro liquids where the second nucleic acid to be detected is located can generate signals. If a plurality of micro liquids overlap, signals emitted from such micro liquids are invalid signals; and signals emitted from single micro liquids that do not overlap are valid signals.

In order to better distinguish the second mixed signal, the first mixed signal and the second signal, t1 and t2 differ by 4°C or above, e.g., by 4-40°C, e.g., by 4-30°C, 4-20°C, 4-18°C, 4-15°C, 4-12°C, 4-10°C, 4-8°C, 4-6°C, 4-5°C, or 4°C. t1 and t3 differ by 4°C or above, e.g., by 4-40°C, e.g., by 4-30°C, 4-20°C, 4-18°C, 4-15°C, 4-12°C, 4-10°C, 4-8°C, 4-6°C, 4-5°C, or 4°C.

In order to obtain a more accurate first signal and third signal, when the second mixed signal, the first mixed signal and the second signal are acquired, the plurality of micro liquids are flatly laid at the bottom of the reaction liquid containing part and always maintain the same position state.

When the signal detection part detects signals generated from the micro liquids through the above controls of the control part, the numbers of classes of target nucleic acids contained in signals obtained from two adjacent signal acquisitions differ by one (i.e., the second mixed signal contains the signals of the first material to be detected, the second material to be detected and the third material to be detected, the first mixed signal contained the signals of the first material to be detected and the second material to be detected, but the second signal only contains the signal of the first material to be detected).

In order to obtain the types and number of the target nucleic acids in the sample to be detected, and realize multiplex detection of different nucleic acids to be detected in the sample to be detected, the signal analysis part performs the following operations.

A third signal is worked out according to the first mixed signal and the second mixed signal acquired by the signal detection part. The signal analysis part subtracts first mixed signal from the second mixed signal to obtain the third signal. Specifically, the signal analysis part subtracts the first mixed signal generated from the micro liquids containing the first material to be detected and the micro liquids containing the second material to be detected at the same position from the second mixed signal generated from the micro liquids to obtain the third signal. The second mixed signal iss a fluorescence signal generated from the micro liquids containing the first material to be detected, the micro liquids containing the second material to be detected and the micro liquids containing the third material to be detected; the first mixed signal is a fluorescence signal generated from the micro liquids containing the first material to be detected and the micro liquids containing the second material to be detected; and the third signal is a fluorescence signal generated from the micro liquids containing the third material to be detected.

A first signal is worked out according to the first mixed signal and the second signal acquired by the signal detection part. The signal analysis part subtracts the second signal from the first mixed signal to obtain the first signal. Specifically, the signal analysis part subtracts the fluorescence signal generated from the micro liquids containing the second material to be detected at the same position from the first mixed signal generated from the micro liquids to obtain the first signal. The first signal is a fluorescence signal generated from the micro liquids containing the first material to be detected, the second signal is a fluorescence signal generated from the micro liquids containing the second material to be detected, and the first mixed signal is a fluorescence signal generated from the micro liquids containing the first material to be detected and the micro liquids containing the second material to be detected.

According to the second signal acquired by the signal detection part, the fluorescence signal generated from the micro liquids containing the second material to be detected may be directly obtained.

Since a single nucleic acid molecule to be detected is distributed into one micro liquid, that is, there is at most one nucleic acid molecule to be detected in one micro liquid (some micro liquids contain no nucleic acid molecules to be detected and thus generate no detectable signals at each signal acquisition temperature), this nucleic acid molecule to be detected may be the first nucleic acid to be detected and may also be the second nucleic acid to be detected or the third nucleic acid to be detected. By analyzing the number of the micro liquids generating signals in the second mixed signal, the total number of the first material to be detected, the second material to be detected and the third material to be detected is obtained; by analyzing the number of the micro liquids generating signals in the first mixed signal, the total number of the first material to be detected and the second material to be detected is obtained; by analyzing the number of micro liquids generating signals in the second signal, the number of the second material to be detected is obtained; by subtracting the total number of the first material to be detected and the second material to be detected generating the first mixed signal from the total number of the first material to be detected, the second material to be detected and the third material to be detected generating the second mixed signal, the number of the third material to be detected is obtained; by subtracting the number of the second material to be detected generating the second signal from the total number of the first material to be detected and the second material to be detected generating the first mixed signal, the number of the first material to be detected is obtained.

### Embodiment 3

The embodiment exemplarily describes a device and method for dual detection: a sample to be detected contains a fourth material to be detected (a fourth target nucleic acid to be detected) and a fifth material to be detected (a fifth target nucleic acid to be detected). It should be noted that a fourth associated product to be detected generated from the fourth material to be detected (the target nucleic acid to be detected undergoes operations such as polymerase chain reaction (PCR) and/or nucleic acid hybridization and/or nucleic acid enzyme digestion to generate the corresponding associated product to be detected, and under normal conditions, the associated product to be detected is a nucleic acid) may bind to a marking substance in a reagent; and similarly, a fifth associated product to be detected generated from the fifth material to be detected may also bind to the marking substance in the reagent. In the embodiment, in a certain temperature mode, the fourth associated product to be detected is generated from the fourth material to be detected and binds to the marking substance, and at the same time, in this temperature mode, the fifth associated product to be detected is generated from the fifth material to be detected and binds to the marking substance. However, the fourth associated product to be detected and the fifth associated product to be detected have different separation temperatures. The fourth associated product to be detected may be separated from the marking substance in the reagent within a fourth separation temperature range, and the fifth associated product to be detected may be separated from the marking substance in the reagent within a fifth separation temperature range. The fourth separation temperature range and the fifth separation temperature range have different minimum temperatures. In the embodiment, the minimum temperature of the fourth separation temperature range is greater than that of the fifth separation temperature range. For example, the fourth associated product to be detected may be separated from the marking substance at a temperature greater than or equal to a temperature t4, and the fifth associated product to be detected may be separated from the marking substance at a temperature greater than or equal to a temperature t5, wherein the temperature t4 is greater than the temperature t5.

In order to realize multiplex detection of different target nucleic acids to be detected in the sample to be detected, and distinguish the different targets (target nucleic acids) by distinguishing the presence or absence of fluorescence through a finite number of photographs, in the embodiment, a mixed liquid of the sample to be detected and the reaction reagent (containing a primer-probe composition and an amplification reagent) is added to the reaction liquid containing part to form a plurality of micro liquid, and the control part performs the following controls.

The control part controls the temperature adjustment part to adjust the temperature of the micro liquids in the reaction liquid containing part, so that the target nucleic acids to be detected in the sample to be detected generate associated products to be detected associated therewith (the target nucleic acids to be detected contain the fourth nucleic acid to be detected and the fifth nucleic acid to be detected, but are presented in different micro liquids); and in this process, the micro liquids may undergo a plurality of temperature cycles, in each of which the micro liquids will change to a plurality of different temperatures and maintain at this temperature for a certain time, and there may be a plurality of cycles, e.g., 20-60, e.g., about 40. In each cycle, the micro liquids will reach a high-temperature state, double-stranded nucleic acids become single-stranded nucleic acids, then the micro liquids reach a low-temperature state, and the single-stranded nucleic acids undergo annealing and extending processes (annealing and extending may also be performed at different temperatures respectively, and the extending temperature is greater than the annealing temperature). After the plurality of temperature cycles, the numbers of the different target nucleic acids to be detected and/or the different associated products to be detected in the sample to be detected are increased (i.e., the quantities of the target nucleic acids to be detected and/or the associated products to be detected in each micro liquid are increased, but the types of the nucleic acids to be detected remain unchanged), which is easier to detect. By reasonably setting the annealing temperature in the amplification process, the target nucleic acids to be detected and/or the associated products to be detected are increased in number, and bind to the marking substance in the reagent, so that the plurality of micro liquids containing the fourth material to be detected or the fifth material to be detected simultaneously generate signals.

Before performing 40 temperature cycles, the micro liquids may be heated to a temperature t0, so that the nucleic acids to be detected in the sample are initially denaturated for a period of time (about 2-5 min). Initial denaturation for a period of time is designed to change the target nucleic acids from a double-stranded state into a single-stranded state.

After completing the 40 temperature cycles, the control part controls the temperature adjustment part to adjust the temperature of the micro liquids to be t4, a plurality of micro liquids only containing the fourth material to be detected generate a signal to form a fourth signal. At the temperature t4, the fifth material to be detected or the associated product thereof is separated from the marking substance, thus generating no detectable signals. However, the fourth material to be detected or the associated product thereof still maintains in a state of binding to the marking substance, thus generating a signal. Similarly, since a single nucleic acid molecule is distributed into one micro liquid, that is, there is only one nucleic acid molecule in one micro liquid, this nucleic acid molecule to be detected may be the fourth nucleic acid to be detected and may also be the fifth nucleic acid to be detected, at the temperature t4, only the plurality of micro liquids where the fourth nucleic acid to be detected is located can generate signals. If a plurality of micro liquids overlap, signals emitted from such micro liquids are invalid signals; and signals emitted from single micro liquids that do not overlap are valid signals.

Next, the control part controls the temperature adjustment part to adjust the temperature of the micro liquids to be t5 (t5 < t4), and a plurality of micro liquids containing the fourth material to be detected and a plurality of micro liquids containing the fifth material to be detected generate signals to form a third mixed signal. At the temperature t5, the fourth material to be detected or the associated product thereof is in a state of binding to the marking substance, thus generating a signal, and the fifth material to be detected or the associated product thereof is also in a state of binding to the marking substance, thus generating a signal. Since a single nucleic acid molecule to be detected is distributed into one micro liquid, that is, there is only one nucleic acid molecule to be detected in one micro liquid, this nucleic acid molecule to be detected may be the fourth nucleic acid to be detected and may also be the fifth nucleic acid to be detected, at the temperature t5, both the micro liquids containing the fourth nucleic acid to be detected and the micro liquids containing the fifth nucleic acid to be detected can generate signals. If a plurality of micro liquids overlapped, signals emitted from such micro liquids were invalid signals; and signals emitted from single micro liquids that do not overlap are valid signals.

In order to better distinguish the fourth signal and the third mixed signal, t4 and t5 differ by 4°C or above, e.g., by 4-40°C, e.g., by 4-30°C, 4-20°C, 4-18°C, 4-15°C, 4-12°C, 4-10°C, 4-8°C, 4-6°C, 4-5°C, or 4°C.

In order to obtain a more accurate fifth signal, when the fourth signal and the third mixed signal are acquired, the plurality of micro liquids are flatly laid at the bottom of the reaction liquid containing part and always maintain the same position state.

When the signal detection part detects signals generated from the micro liquids through the above controls of the control part, the numbers of classes of target nucleic acids contained in signals obtained from two adjacent signal acquisitions differ by one (i.e., the fourth signal only contains the signal of the fourth material to be detected, but the third mixed signal contains the signals of the fourth material to be detected and the fifth material to be detected).

In order to obtain the types and number of the target nucleic acids in the sample to be detected, and realize multiplex detection of different nucleic acids to be detected in the sample to be detected, the signal analysis part performs the following operations.

A fifth signal is worked out according to the fourth signal and the third mixed signal acquired by the signal detection part. The signal analysis part subtracts the fourth signal from the third mixed signal to obtain the fifth signal. Specifically, the signal analysis part subtracts the fluorescence signal generated from the micro liquids containing the fourth material to be detected at the same position from the third mixed signal generated from the micro liquids to obtain the fifth signal. The fourth signal is a fluorescence signal generated from the micro liquids containing the fourth material to be detected, the fifth signal is a fluorescence signal generated from the micro liquids containing the fifth material to be detected, and the third mixed signal is a fluorescence signal generated from the micro liquids containing the fourth material to be detected and the micro liquids containing the fifth material to be detected.

According to the fourth signal acquired by the signal detection part, the fluorescence signal generated from the micro liquids containing the fourth material to be detected may be directly obtained.

Since a single nucleic acid molecule to be detected is distributed into one micro liquid, that is, there is at most one nucleic acid molecule to be detected in one micro liquid (some micro liquids contain no nucleic acid molecules to be detected and thus generate no signals at each signal acquisition temperature), this nucleic acid molecule to be detected may be the fourth nucleic acid to be detected and may also be the fifth nucleic acid to be detected. By analyzing the number of the micro liquids generating signals in the third mixed signal, the total number of the fourth material to be detected and the fifth material to be detected is obtained; by analyzing the number of the micro liquids generating signals in the fourth signal, the number of the fourth material to be detected is obtained; and by subtracting the number of the fourth material to be detected generating the fourth signal from the total number of the fourth material to be detected and the fifth material to be detected generating the third mixed signal, the number of the fifth material to be detected is obtained.

Certainly, a person skilled in the art may, according to the above embodiments, reasonably infer how to use the technical solutions of the present application to realize time-saving and efficient quadruple nucleic acid detection (detection of four kinds of target nucleic acids in the sample to be detected), quintuple nucleic acid detection (detection of five kinds of target nucleic acids in the sample to be detected), sextuple nucleic acid detection (detection of six kinds of target nucleic acids in the sample to be detected), or more multiple nucleic acid detection.

A person of ordinary skill in the art may understand that all or part of the processes in the methods of the above embodiments may be implemented by instructing relevant hardware through a computer program. The program may be stored in a non-volatile computer-readable storage medium, and when the program is executed, the processes in the method of the above method embodiments may be included. The storage medium may be a magnetic disk, an optical disk, an ROM, etc.

Any reference to a memory, a storage, a database, or other mediums as used herein may include a non-volatile and/or volatile memory. Suitable non-volatile memory may include an ROM, a Programmable ROM (PROM), an Erasable PROM (EPROM), an Electrically Erasable PROM (EEPROM), or a flash memory. The volatile memory may include a random access memory (RAM), which is used as an external cache memory. As an illustration rather than a limitation, the RAM may have multiple forms, such as a Static RAM (SRAM), a Dynamic Random Access Memory (DRAM), a synchronous DRAM (SDRAM), a Double Data Rate SDRAM (DDR SDRAM), an Enhanced Synchronous DRAM (ESDRAM), a Synchlink DRAM (SLDRAM), a Rambus DRAM (RDRAM) and a Direct Rambus DRAM (DRDRAM).

It should be understood that "one example ( 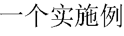 )" or "one embodiment ( 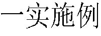 )" mentioned throughout the description means that specific characteristics, structures or features related to the embodiment/example are included in at least one embodiment/example of the present application. Therefore, "in one example ( 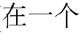 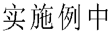 )" or "in one embodiment ( 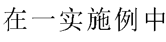 )" appearing throughout the description does not necessarily refer to the same embodiment/example. In addition, these specific characteristics, structures or features may be combined in one or more embodiments/examples in any suitable manners. A person skilled in the art should also be aware that all the embodiments/examples described in the description are optional embodiments/examples, and the actions and modules involved are not necessarily required by the present application.

In various embodiments/examples of the present application, it should be understood that the magnitude of the serial number of each of the above processes does not mean an inevitable sequence of the order of execution, and the order of execution of each process should be determined by its function and inherent logic without constituting any limitation on the implementation process of the embodiments/examples of the present application.

The functional units in the embodiments/examples of the present application may be integrated in one processing unit, or each unit may physically exist separately, or two or more units may be integrated in one unit. The above integrated unit may be implemented either in the form of hardware or in the form of a software function unit.

The above integrated unit may be stored in a computer-accessible memory if it is implemented in the form of the software function unit and sold or used as an independent product. Based on such an understanding, the technical solutions of the present application essentially, or the part contributing to the prior art, or all or part of the technical solutions may be presented in the form of a software product. The computer software product is stored in a memory including several requests to enable a computer device (which may be a personal computer, a server, a network device, or the like) to perform part or all of the steps of the methods described in the embodiments/examples of the present application.

A device for detecting multiple target nucleic acids and a detection method according to the embodiments/examples of the present application are introduced above in details. The principles and implementations of the present application are set forth herein with specific examples. The description of the above embodiments/examples is only for the purpose of assisting in understanding the method and core concept of the present application. In the meantime, for a person of general skill in the art, according to the idea of the present application, there will be changes in the specific implementations and the scope of application. To sum up, the contents of the description should not be understood as a limitation of the present application.

## Claims

1. A method for detecting multiple target nucleic acids, comprising the following contents:
mixing a primer-probe composition, a sample to be detected and an amplification reagent to obtain a reaction system; the amplification reagent comprising a nucleic acid polymerase and dNTPs;
placing the reaction system in a condition that allows the nucleic acid polymerase to perform hybridization and extension reaction, so as to obtain a reaction product;
placing the reaction product at n different signal acquisition temperatures for a total of n signal acquisitions with each signal acquisition temperature for one signal acquisition; each signal acquisition comprising at least one signal channel acquisition;
analyzing whether there is a difference between signals obtained from two adjacent signal channel acquisitions under a same signal channel to determine presence or absence of the target nucleic acids in the sample to be detected; and/or
analyzing presence or absence of a signal obtained from a signal channel acquisition with a maximum signal acquisition temperature under the same signal channel to determine the presence or absence of the target nucleic acids in the sample to be detected;
wherein n is an integer ≥ 2, and n ≤ a number of classes of the target nucleic acids.

2. The method for detecting multiple target nucleic acids according to claim 1, **characterized in that** numbers of classes of target nucleic acids contained in the signals obtained from the two adjacent signal channel acquisitions under the same signal channel differ by one at most; preferably, the numbers of classes of the target nucleic acids contained in the signals obtained from the two adjacent signal channel acquisitions under the same signal channel differ by one; and preferably, the signal obtained from the signal channel acquisition with the maximum signal acquisition temperature contains at most one kind of target nucleic acid.

3. The method for detecting multiple target nucleic acids according to claim 1, **characterized in that** signal acquisition temperatures used for the two adjacent signal channel acquisitions under the same signal channel differ by 4°C or above.

4. The method for detecting multiple target nucleic acids according to claim 1, **characterized in that** each signal acquisition comprises m signal channel acquisitions; and m refers to a number of classes of detection labels of a probe in the primer-probe composition.

5. The method for detecting multiple target nucleic acids according to claim 1, **characterized in that** before the placing the reaction system in a condition that allows the nucleic acid polymerase to perform hybridization and extension reaction, the method further comprises: distributing the reaction system into 500 or more reaction units, with each reaction unit containing one target nucleic acid of the sample to be detected or containing no target nucleic acids of the sample to be detected; preferably, the signal acquisitions refer to acquisitions of fluorescence signals by means of a camera; and the signal channel acquisitions refer to acquisitions of the fluorescence signals by means of the camera under a fluorescence signal channel.

6. The method for detecting multiple target nucleic acids according to claim 1, **characterized in that** the condition that allows the nucleic acid polymerase to perform hybridization and extension reaction comprises: initial denaturation at about 85°C to about 105°C for 0 to about 15 min; denaturation at about 85°C to about 105°C for about 1 to about 60 s, and annealing and extending at about 40°C to about 75°C for about 3 to about 90 s, for 20 to 60 cycles; preferably, when the target in the sample to be detected is RNA, the amplification reagent further comprises a reverse transcriptase, and a reaction condition for a first PCR amplification of the reaction system comprises: reverse transcription at about 30 to about 65°C for about 2 to about 30 min; initial denaturation at about 85°C to about 105°C for 0 to about 15 min; denaturation at about 85°C to about 105°C for about 1 to about 60 s, and annealing and extending at about 40°C to about 75°C for about 3 to about 90 s, for 20 to 60 cycles.

7. The method for detecting multiple target nucleic acids according to claim 1, **characterized in that** the primer-probe composition comprises a first probe and a first primer mixture;
the first primer mixture comprises at least two kinds of primer sets, and the different kinds of primer sets specifically bind to different kinds of target nucleic acids respectively; the primer sets in the first primer mixture specifically bind to the corresponding target nucleic acids to generate a pre-product, the pre-product contains a single-stranded pre-product specifically binding to the first probe, and the single-stranded pre-product specifically binds to the first probe and extends by >_ 0 base to form a double-stranded product, and formation of the double-stranded product causes a detectable signal change;
preferably, the different kinds of primer sets in the first primer mixture and the corresponding target nucleic acids generate different single-stranded pre-products, the different single-stranded pre-products and the first probe form different double-stranded products, and the different double-stranded products have different melting temperatures; and preferably, the melting temperatures of the different double-stranded products differ by 4°C or above.

8. The method for detecting multiple target nucleic acids according to claim 6, **characterized in that** the primer-probe composition further comprises a second probe and a second primer mixture;
the second probe and the first probe have different base sequences and are modified with different detection labels; the second primer mixture comprises at least one kind of primer set, and different kinds of primer sets specifically bind to different kinds of target nucleic acids respectively; preferably, the primer sets in the second primer mixture specifically bind to the corresponding target nucleic acids to generate pre-products, the pre-products contains single-stranded pre-products specifically binding to the second probe, and the single-stranded pre-products specifically bind to the second probe and extend by >_ 0 base to form double-stranded products, and formation of the double-stranded product causes a detectable signal change;
preferably, the different kinds of primer sets in the second primer mixture and the corresponding target nucleic acids generate different single-stranded pre-products, the different single-stranded pre-products and the second probe form different double-stranded products, and the different double-stranded products have different melting temperatures; and preferably, the melting temperatures of the different double-stranded products differ by 4°C or above.

9. The method for detecting multiple target nucleic acids according to claim 7 or 8, **characterized in that** the first probe or the second probe is a sequence that does not specifically bind to any target nucleic acids, and comprises a probe signal detection region (H), and sequences of the probe signal detection regions (H) of the different probes are different from each other;
each primer set comprises a first primer and a second primer, and the first primer contains target sequence binding regions 1; the second primer contains primer signal detection regions (h) and target sequence binding regions 2, and the primer signal detection regions (h) are located at 5' ends of the target sequence binding regions 2; each primer signal detection region (h) is a sequence that does not specifically bind to any target nucleic acids and partially or wholly identical to the probe signal detection region (H) of the corresponding probe; sequences of the primer signal detection regions (h) of the second primers in the different primer sets are different from each other;
preferably, the first probe or the second probe further comprises a primer anchoring region (A'); the first primer of the at least one kind of primer set in the first primer mixture or the second primer mixture further comprises a probe anchoring region (A), and the probe anchoring region (A) is located at a 5' end of the target sequence binding region 1; the probe anchoring region (A) does not specifically bind to any target nucleic acids but specifically binds to the primer anchoring region (A');
preferably, in the first primer mixture or the second primer mixture, the second primer of at most one kind of primer set further comprises an extension blocking region (M), the extension blocking region (M) is located at a 5' end of the primer signal detection region (h), and neither the extension blocking region (M) nor a complementary sequence thereof specifically binds to any probe or any target nucleic acids.

10. A device for detecting multiple target nucleic acids, comprising:
a reaction liquid containing part, configured to contain a plurality of micro liquids, each micro liquid containing a reaction reagent, and part of the micro liquids further containing one of a first material to be detected or a second material to be detected;
a temperature adjustment part, configured to adjust a temperature of the micro liquids in the reaction liquid containing part;
a signal detection part, configured to detect a signal generated from the micro liquids in the reaction liquid containing part;
a control part, the control part controlling the temperature adjustment part to adjust the temperature of the micro liquids in the reaction liquid containing part in such a way that the plurality of micro liquids containing the first material to be detected or the second material to be detected simultaneously generate the signal;
the control part controlling the temperature adjustment part to adjust the temperature of the micro liquids to be 11, a plurality of micro liquids containing the first material to be detected and a plurality of micro liquids containing the second material to be detected generate signals to form a first mixed signal;
the control part controlling the temperature adjustment part to adjust the temperature of the micro liquids to be t2, the plurality of micro liquids only containing the second material to be detected generate a signal to form a second signal;
wherein t1 < t2; the control part controlling the signal detection part to acquire, at the temperatures t1 and t2, the signals generated from the micro liquids and output the first mixed signal and the second signal; and
a signal analysis part, the signal analysis part working out a first signal according to the first mixed signal and the second signal acquired by the signal detection part.

11. The device for detecting multiple target nucleic acids according to claim 10, **characterized in that** the control part controls the temperature adjustment part to adjust the temperature of the micro liquids in the reaction liquid containing part to be t3, the plurality of micro liquids containing the first material to be detected, the plurality of micro liquids containing the second material to be detected and a plurality of micro liquids containing a third material to be detected generate signals to form a second mixed signal, wherein t3 < t1, and t3 and t1 differ by 4°C or above.

12. The device for detecting multiple target nucleic acids according to claim 10 or 11, **characterized in that** t1 and t2 differ by 4°C or above.

13. The device for detecting multiple target nucleic acids according to claim 10 or 11, **characterized in that** t1 and t2 differ by 4°C.

14. The device for detecting multiple target nucleic acids according to claim 10 or 11, **characterized in that** the signal analysis part subtracts the second signal from the first mixed signal to obtain the first signal.

15. The device for detecting multiple target nucleic acids according to claim 10 or 11, **characterized in that** when the first mixed signal and the second signal are acquired, the plurality of micro liquids are flatly laid at a bottom of the reaction liquid containing part and always maintain a same position state.

16. The device for detecting multiple target nucleic acids according to claim 14 or 15, **characterized in that** the first signal is a fluorescence signal generated from the micro liquids containing the first material to be detected, the second signal is a fluorescence signal generated from the micro liquids containing the second material to be detected, and the first mixed signal is a fluorescence signal generated from the micro liquids containing the first material to be detected and the micro liquids containing the second material to be detected.

17. The device for detecting multiple target nucleic acids according to claim 16, **characterized in that** the signal analysis part subtracts the fluorescence signal generated from the micro liquids containing the second material to be detected at a same position from the first mixed signal generated from the micro liquids to obtain the first signal.

18. The device for detecting multiple target nucleic acids according to claim 11, **characterized in that** the signal analysis part subtracts the first mixed signal generated from the micro liquids containing the first material to be detected and the micro liquids containing the second material to be detected at a same position from the second mixed signal generated from the micro liquids to obtain a third signal, and the third signal is a fluorescence signal generated from micro liquids containing the third material to be detected.
